(19) Europäisches Patentamt
European Patent Office
Office européen des brevets

(11) EP 3 269 729 B1

(12) EUROPEAN PATENT SPECIFICATION

(45) Date of publication and mention of the grant of the patent:
**21.08.2019 Bulletin 2019/34**

(51) Int Cl.:
*C07K 14/32* (2006.01) *C11D 3/38* (2006.01)

(21) Application number: **16179550.5**

(22) Date of filing: **14.07.2016**

# (54) DETERGENT COMPOSITION

REINIGUNGSZUSAMMENSETZUNG

COMPOSITION DE NETTOYAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**17.01.2018 Bulletin 2018/03**

(73) Proprietor: **The Procter & Gamble Company**
**Cincinnati, OH 45202 (US)**

(72) Inventors:
- **LANT, Neil Joseph**
  **Newcastle upon Tyne, NE12 9TS (GB)**
- **BETTIOL, Jean-Luc Philippe**
  **1853 Strombeek-Bever (BE)**
- **GONZALES, Denis Alfred**
  **1853 Strombeek-Bever (BE)**
- **HAYWARD, Adam Simon**
  **Newcastle upon Tyne, NE12 9TS (GB)**

(74) Representative: **Siddiquee, Sanaul Kabir**
**N.V. Procter & Gamble**
**Services Company S.A.**
**Temselaan 100**
**1853 Strombeek-Bever (BE)**

(56) References cited:
**WO-A1-2016/027078 US-A1- 2015 191 676**

- **KAZUO KOBAYASHI ET AL: "BslA(YuaB) forms a hydrophobic layer on the surface of Bacillus subtilis biofilms", MOLECULAR MICROBIOLOGY., vol. 85, no. 1, 28 May 2012 (2012-05-28), pages 51-66, XP055333868, GB ISSN: 0950-382X, DOI: 10.1111/j.1365-2958.2012.08094.x**
- **Laura Hobley ET AL: "BslA is a self-assembling bacterial hydrophobin that coats the Bacillus subtilis biofilm", Proceedings of the National Academy of Sciences, 13 August 2013 (2013-08-13), pages 13600-13605, XP055235003, United States DOI: 10.1073/pnas.1306390110 Retrieved from the Internet: URL:http://www.pnas.org/content/110/33/13600.full.pdf [retrieved on 2015-12-09]**
- **KEITH M. BROMLEY ET AL: "Interfacial self-assembly of a bacterial hydrophobin", PROCEEDINGS OF THE NATIONAL ACADEMY OF SCIENCES, vol. 112, no. 17, 13 April 2015 (2015-04-13), pages 5419-5424, XP055237000, US ISSN: 0027-8424, DOI: 10.1073/pnas.1419016112**
- **GIOVANNI B. BRANDANI ET AL: "The Bacterial Hydrophobin BslA is a Switchable Ellipsoidal Janus Nanocolloid", LANGMUIR, vol. 31, no. 42, 27 October 2015 (2015-10-27), pages 11558-11563, XP055237002, US ISSN: 0743-7463, DOI: 10.1021/acs.langmuir.5b02347**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> The Procter & Gamble Company

<120> Detergent Composition

<130> CM04542F

<160> 8

<170> PatentIn version 3.5

<210> 1
<211> 181
<212> PRT
<213> Bacillus subtilis

<400> 1

```
Met Lys Arg Lys Leu Leu Ser Ser Leu Ala Ile Ser Ala Leu Ser Leu
1               5                   10                  15

Gly Leu Leu Val Ser Ala Pro Thr Ala Ser Phe Ala Ala Glu Ser Thr
            20                  25                  30

Ser Thr Lys Ala His Thr Glu Ser Thr Met Arg Thr Gln Ser Thr Ala
        35                  40                  45

Ser Leu Phe Ala Thr Ile Thr Gly Ala Ser Lys Thr Glu Trp Ser Phe
    50                  55                  60

Ser Asp Ile Glu Leu Thr Tyr Arg Pro Asn Thr Leu Leu Ser Leu Gly
65                  70                  75                  80

Val Met Glu Phe Thr Leu Pro Ser Gly Phe Thr Ala Asn Thr Lys Asp
                85                  90                  95

Thr Leu Asn Gly Asn Ala Leu Arg Thr Thr Gln Ile Leu Asn Asn Gly
            100                 105                 110

Lys Thr Val Arg Val Pro Leu Ala Leu Asp Leu Leu Gly Ala Gly Glu
        115                 120                 125

Phe Lys Leu Lys Leu Asn Asn Lys Thr Leu Pro Ala Ala Gly Thr Tyr
    130                 135                 140

Thr Phe Arg Ala Glu Asn Lys Ser Leu Ser Ile Gly Asn Lys Phe Tyr
145                 150                 155                 160

Ala Glu Ala Ser Ile Asp Val Ala Lys Arg Ser Thr Pro Pro Thr Gln
                165                 170                 175

Pro Cys Gly Cys Asn
            180
```

<210> 2
<211> 181
<212> **PRT**
<213> Bacillus licheniformis

<400> 2

```
Met Lys Met Lys His Lys Phe Phe Ser Thr Val Met Ala Ser Leu Phe
1               5                   10                  15

Gly Leu Val Leu Leu Leu Ser Leu Pro Thr Ala Ser Phe Ala Ala Glu
            20                  25                  30

Ser Ser Ser Thr Val His Glu Pro Glu Met Ser Thr Lys Ala Thr Ala
        35                  40                  45

Thr Leu Phe Ala Lys Tyr Thr Gly Ala Ser Gln Gln Glu Trp Ser Phe
    50                  55                  60

Ser Asp Ile Glu Leu Thr Tyr Arg Pro Asn Thr Ile Leu Ser Leu Gly
65                  70                  75                  80

Val Met Glu Phe Thr Leu Pro Ser Gly Phe Thr Ala Thr Thr Lys Asp
                85                  90                  95

Thr Val Asn Gly His Ala Leu Arg Glu Arg Gln Ile Leu Asn Asn Gly
            100                 105                 110

Lys Thr Val Arg Leu Pro Leu Asn Ile Asp Leu Ile Gly Ala Ala Glu
        115                 120                 125

Phe Lys Leu Ser Leu Asn Asn Lys Thr Leu Pro Ala Ala Gly Thr Tyr
    130                 135                 140

Lys Phe Arg Ala Glu Asn Lys Ser Leu Ser Ile Gly Ser Lys Phe Tyr
145                 150                 155                 160

Ala Glu Asp Thr Ile Val Val Gln Lys Arg Ser Thr Pro Pro Thr Gln
                165                 170                 175

Pro Cys Asn Cys Lys
            180
```

<210> 3
<211> 180
<212> PRT
<213> Bacillus amyloliquefaciens

<400> 3

```
Met Leu Lys Arg Met Tyr Arg Ser Lys Leu Ser Ile Leu Ala Val Ser
1               5                   10                  15

Leu Val Met Met Val Ser Ile Phe Leu Pro Ser Phe Gln Ala Ser Ala
            20                  25                  30

Gln Thr Thr Lys Thr Glu Ser Val Tyr Arg Pro Ala Ala Asn Ala Ser
        35                  40                  45

Leu Tyr Ala Thr Ile Thr Gly Ala Ser Lys Gln Glu Trp Ser Phe Ser
    50                  55                  60

Asp Ile Glu Leu Thr Tyr Arg Pro Asn Ser Ile Leu Ala Leu Gly Thr
65                  70                  75                  80

Val Glu Phe Thr Leu Pro Ser Gly Phe Ser Ala Thr Thr Lys Asp Thr
                85                  90                  95

Val Asn Gly Arg Ala Leu Thr Thr Gly Gln Ile Leu Asn Asn Gly Lys
            100                 105                 110

Thr Val Arg Leu Pro Leu Thr Ile Asp Leu Leu Gly Ile Ala Glu Phe
        115                 120                 125

Lys Leu Val Leu Ala Asn Lys Thr Leu Pro Ala Ala Gly Lys Tyr Thr
    130                 135                 140

Phe Arg Ala Glu Asn Arg Val Leu Gly Leu Gly Ser Thr Phe Tyr Ala
145                 150                 155                 160

Glu Ser Ser Ile Glu Val Gln Lys Arg Ala Thr Pro Pro Thr Gln Pro
                165                 170                 175

Cys Asn Cys Lys
            180
```

<210> 4
<211> 177
<212> **PRT**
<213> Bacillus pumilus

<400> 4

```
Met Lys Lys Thr Trp Thr Met Ile Met Met Gly Met Leu Thr Leu Val
1               5                   10                  15

Met Ala Leu Ser Val Pro Ile Ala Ala Ser Ala Glu Gly Ala Thr Gln
```

20 25 30

Glu Gly Lys Ala Ser Thr Asn Ala Arg Pro Ala Glu Leu Tyr Ala Lys
35 40 45

Ile Thr Gly Thr Ser Lys Gln Glu Trp Ser Phe Ser Asp Ile Glu Leu
50 55 60

Thr Tyr Arg Pro Asn Ser Val Leu Ser Leu Gly Ala Ile Glu Phe Thr
65 70 75 80

Leu Pro Ala Gly Phe Gln Ala Thr Thr Lys Asp Ile Phe Asn Gly Lys
85 90 95

Ala Leu Lys Asp Ser Tyr Ile Leu Asn Ser Gly Lys Thr Val Arg Ile
100 105 110

Pro Ala Arg Leu Asp Leu Leu Gly Ile Ser Gln Phe Lys Leu Gln Leu
115 120 125

Ser His Lys Val Leu Pro Ala Ala Gly Thr Tyr Thr Phe Arg Ala Glu
130 135 140

Asn Arg Ala Leu Ser Ile Gly Ser Lys Phe Tyr Ala Glu Asp Thr Leu
145 150 155 160

Asp Ile Gln Thr Arg Pro Val Val Val Thr Pro Pro Asp Pro Cys Gly
165 170 175

Cys

<210> 5
<211> 154
<212> **PRT**
<213> Bacillus subtilis

<400> 5

```
Met Leu Lys Arg Thr Ser Phe Val Ser Ser Leu Phe Ile Ser Ser Ala
1               5                   10                  15

Val Leu Leu Ser Ile Leu Leu Pro Ser Gly Gln Ala His Ala Gln Ser
            20                  25                  30

Ala Ser Ile Glu Ala Lys Thr Val Asn Ser Thr Lys Glu Trp Thr Ile
        35                  40                  45

Ser Asp Ile Glu Val Thr Tyr Lys Pro Asn Ala Val Leu Ser Leu Gly

    50                  55                  60

Ala Val Glu Phe Gln Phe Pro Asp Gly Phe His Ala Thr Thr Arg Asp
65                  70                  75                  80

Ser Val Asn Gly Arg Thr Leu Lys Glu Thr Gln Ile Leu Asn Asp Gly
                85                  90                  95

Lys Thr Val Arg Leu Pro Leu Thr Leu Asp Leu Leu Gly Ala Ser Glu
            100                 105                 110

Phe Asp Leu Val Met Val Arg Lys Thr Leu Pro Arg Ala Gly Thr Tyr
        115                 120                 125

Thr Ile Lys Gly Asp Val Val Asn Gly Leu Gly Ile Gly Ser Phe Tyr
    130                 135                 140

Ala Glu Thr Gln Leu Val Ile Asp Pro Arg
145                 150
```

<210> 6
<211> 140
<212> PRT
<213> Bacillus subtilis

<400> 6

```
Met Arg Thr Gln Ser Thr Ala Ser Leu Phe Ala Thr Ile Thr Gly Ala
1               5                   10                  15

Ser Lys Thr Glu Trp Ser Phe Ser Asp Ile Glu Leu Thr Tyr Arg Pro
            20                  25                  30

Asn Thr Leu Leu Ser Leu Gly Val Met Glu Phe Thr Leu Pro Ser Gly
        35                  40                  45

Phe Thr Ala Asn Thr Lys Asp Thr Leu Asn Gly Asn Ala Leu Arg Thr
    50                  55                  60

Thr Gln Ile Leu Asn Asn Gly Lys Thr Val Arg Val Pro Leu Ala Leu
65                  70                  75                  80

Asp Leu Leu Gly Ala Gly Glu Phe Lys Leu Lys Leu Asn Asn Lys Thr
                85                  90                  95

Leu Pro Ala Ala Gly Thr Tyr Thr Phe Arg Ala Glu Asn Lys Ser Leu
            100                 105                 110

Ser Ile Gly Asn Lys Phe Tyr Ala Glu Ala Ser Ile Asp Val Ala Lys


              115                 120                 125

      Arg Ser Thr Pro Pro Thr Gln Pro Cys Gly Cys Asn
          130                 135                 140
```

<210> 7
<211> 181
<212> PRT
<213> Bacillus subtilis

<220>
<221> MISC_FEATURE
<222> (178)..(178)
<223> Xaa is any amino acid that is not cysteine or methionine

<220>
<221> MISC_FEATURE
<222> (180)..(180)
<223> Xaa is any amino acid that is not cysteine or methionine

<400> 7

```
Met Lys Arg Lys Leu Leu Ser Ser Leu Ala Ile Ser Ala Leu Ser Leu
1               5                   10                  15

Gly Leu Leu Val Ser Ala Pro Thr Ala Ser Phe Ala Ala Glu Ser Thr
            20                  25                  30

Ser Thr Lys Ala His Thr Glu Ser Thr Met Arg Thr Gln Ser Thr Ala
        35                  40                  45

Ser Leu Phe Ala Thr Ile Thr Gly Ala Ser Lys Thr Glu Trp Ser Phe
    50                  55                  60

Ser Asp Ile Glu Leu Thr Tyr Arg Pro Asn Thr Leu Leu Ser Leu Gly
65                  70                  75                  80

Val Met Glu Phe Thr Leu Pro Ser Gly Phe Thr Ala Asn Thr Lys Asp
                85                  90                  95

Thr Leu Asn Gly Asn Ala Leu Arg Thr Thr Gln Ile Leu Asn Asn Gly
            100                 105                 110

Lys Thr Val Arg Val Pro Leu Ala Leu Asp Leu Leu Gly Ala Gly Glu
        115                 120                 125

Phe Lys Leu Lys Leu Asn Asn Lys Thr Leu Pro Ala Ala Gly Thr Tyr
    130                 135                 140

Thr Phe Arg Ala Glu Asn Lys Ser Leu Ser Ile Gly Asn Lys Phe Tyr
145                 150                 155                 160

Ala Glu Ala Ser Ile Asp Val Ala Lys Arg Ser Thr Pro Pro Thr Gln
                165                 170                 175

Pro Xaa Gly Xaa Asn
            180
```

<210> 8
<211> 181
<212> PRT
<213> Bacillus subtilis

<400> 8

```
Met Lys Arg Lys Leu Leu Ser Ser Leu Ala Ile Ser Ala Leu Ser Leu
1               5                   10                  15

Gly Leu Leu Val Ser Ala Pro Thr Ala Ser Phe Ala Ala Glu Ser Thr
            20                  25                  30

Ser Thr Lys Ala His Thr Glu Ser Thr Met Arg Thr Gln Ser Thr Ala
        35                  40                  45

Ser Leu Phe Ala Thr Ile Thr Gly Ala Ser Lys Thr Glu Trp Ser Phe
    50                  55                  60

Ser Asp Ile Glu Leu Thr Tyr Arg Pro Asn Thr Leu Leu Ser Leu Gly
65                  70                  75                  80

Val Met Glu Phe Thr Leu Pro Ser Gly Phe Thr Ala Asn Thr Lys Asp
                85                  90                  95

Thr Leu Asn Gly Asn Ala Leu Arg Thr Thr Gln Ile Leu Asn Asn Gly
            100                 105                 110

Lys Thr Val Arg Val Pro Leu Ala Leu Asp Leu Leu Gly Ala Gly Glu
        115                 120                 125

Phe Lys Leu Lys Leu Asn Asn Lys Thr Leu Pro Ala Ala Gly Thr Tyr
    130                 135                 140

Thr Phe Arg Ala Glu Asn Lys Ser Leu Ser Ile Gly Asn Lys Phe Tyr
145                 150                 155                 160

Ala Glu Ala Ser Ile Asp Val Ala Lys Arg Ser Thr Pro Pro Thr Gln
                165                 170                 175

Pro Ala Gly Ala Asn
            180
```

## Description

REFERENCE TO A SEQUENCE LISTING

This application contains Sequence Listings in computer readable form. The computer readable form is incorporated herein by reference.

FIELD OF THE INVENTION

The present invention relates to a detergent composition comprising a surfactant system and a BslA-like protein. The composition provides good cleaning, long lasting suds and surface modification that can contribute to second time cleaning benefits, improved drying, improved shine, in the case of dishware, easiness of ironing, in the case of fabrics, reduced biofilm growth, etc.

BACKGROUND OF THE INVENTION

Cleaning compositions should provide good soil and grease cleaning while presenting a good suds profile. Users usually see suds as an indicator of the performance of the cleaning composition. Moreover, the user of a cleaning composition may also use the suds profile and the appearance of the suds (density, whiteness) as an indicator that the wash solution still contains active cleaning ingredients. This is particularly the case for manual washing, also referred to herein as hand-washing, where the user usually doses the cleaning composition depending on the suds remaining and renews the wash solution when the suds subsides or when the suds does not look thick enough. Thus, a cleaning composition, particularly a manual wash cleaning composition that generates little or low density suds would tend to be replaced by the user more frequently than is necessary.

Thus, it is desirable for a cleaning composition to provide good suds height and density as well as good suds duration during the initial mixing of the composition with water and during the entire washing operation. When used in a manual-washing process, the composition should also provides a pleasant washing experience, i.e., good feel on the user's hands during the wash. The composition should also be easy to rinse. It is also desirable that cleaning compositions provide surface modification, contributing to shine, in the case of dishware, easy ironing in the case of fabrics, improved second time cleaning and reduction of biofilm.

There is also the desire to reduce the amount of surfactants without impacting suds. Thus, there is the need to find new compositions that improve cleaning, suds longevity and improved after cleaning benefits in hand washing.

SUMMARY OF THE INVENTION

According to the present invention there is provided a detergent composition comprising a BslA (Biofilm surface layer A) protein and a surfactant system. Surfactants can denature proteins but that does not seem to be the case in the composition of the invention.

The detergent composition is a manual-washing composition. Preferably the detergent composition is for manual dishwashing. Preferably the detergent composition is for laundry manual-washing, preferably for washing delicate fabrics. Preferred compositions are in the form of a liquid, optionally enclosed in a water soluble film in the form of a pouch, preferably a multi-compartment pouch, optionally with a particulate composition in at least one compartment.

The invention also provides a method of washing soiled surfaces comprising forming a wash liquor comprising a surfactant system and a BslA-like protein, contacting the surfaces with the wash liquor, and optionally rinsing and drying the surfaces.

The invention also provides a method of washing soiled surfaces comprising contacting a soiled surface directly with the composition, optionally using a cleaning device, and then contacting the soiled surface and detergent composition with water for cleaning and/or rinsing. The surfaces may be modified by the BslA-like protein by such washing methods, for example resulting in improved soil removal, improved soil release after subsequent soiling, faster drying, improved shine, easier ironing or prevention of biofilm growth.

The composition of the invention provides good cleaning and good suds profile, especially in the presence of greasy soils. It can also provide surface modifications facilitating next time cleaning.

According to the present invention, there is provided a method of manual washing comprising the step of: delivering the detergent composition to a volume of water and immersing soiled articles in the water. When the composition of the invention is used according to this method an excellent suds profile, with a long lasting effect is achieved. Preferably, the concentration of the surfactant system in the wash liquor is less than five times the critical micelle concentration of any of the surfactants of the surfactant system, preferably less than the critical micelle concentration of any of the surfactants of the surfactant system.

According to the present invention, there is provided a method of manual washing comprising the step of: delivering the detergent composition of the invention directly onto soiled articles or onto a cleaning implement and using the cleaning implement to clean the soiled articles. Preferably the cleaning implement is a sponge and more preferably the sponge is wet.

There is also provided the use of a BslA-like protein to confer surface modification during cleaning to provide benefits after cleaning, such as easier next time cleaning, easier ironing, in the case of fabrics, reduction of biofilm growth, etc.

Preferably the manual washing is dishwashing and the soiled articles comprise soiled dishware. As used herein, "dishware" includes cookware and tableware.

BRIEF DESCRIPTION OF THE DRAWINGS

Figure 1 is a plot of the surface tension of a surfactant (mN/m) versus the concentration of the surfactant (mg/l). It shows the inflection point that corresponds to the critical micelle concentration (CMC).

DETAILED DESCRIPTION OF THE INVENTION

Definitions

As used herein, the articles "a" and "an" when used in a claim, are understood to mean one or more of what is claimed or described.

As used herein, the term "substantially free of' or "substantially free from" means that the indicated material is present in an amount of no more than about 5 wt%, preferably no more than about 2%, and more preferably no more than about 1 wt% by weight of the composition.

As used therein, the term "essentially free of' or "essentially free from" means that the indicated material is present in an amount of no more than about 0.1 wt% by weight of the composition, or preferably not present at an analytically detectible level in such composition. It may include compositions in which the indicated material is present only as an impurity of one or more of the materials deliberately added to such compositions.

As used herein the phrase "cleaning composition," "detergent composition," or "detergent or cleaning composition" are used interchangeably herein to refer to compositions and formulations designed for cleaning soiled surfaces. Such compositions include but are not limited to, dishwashing compositions, laundry detergent compositions, fabric softening compositions, fabric enhancing compositions, fabric freshening compositions, laundry prewash, laundry pretreat, laundry additives, spray products, dry cleaning agent or composition, laundry rinse additive, wash additive, post-rinse fabric treatment, ironing aid, dish washing compositions, hard surface cleaning compositions, unit dose formulation, delayed delivery formulation, detergent contained on or in a porous substrate or nonwoven sheet, and other suitable forms that may be apparent to one skilled in the art in view of the teachings herein. Such compositions may be used as a pre-cleaning treatment, a post-cleaning treatment, or may be added during the rinse or wash cycle of the cleaning process. The cleaning compositions may have a form selected from liquid, powder, single-phase or multi-phase unit dose or pouch form, tablet, gel, paste, bar, or flake. In a preferred embodiment of the present invention, the cleaning composition of the present invention comprises a dish detergent composition, which is in a single phase or multiphase unit dose form as encapsulated by a single compartment or multi-compartment water-soluble pouch, e.g., formed by a water-soluble polymer such as polyvinyl alcohol (PVA) or copolymers thereof. Preferably the composition is for manual-washing. The cleaning composition of the present invention is a dishwashing detergent. Preferably the composition is in the form of a liquid.

As used herein the term "increased suds longevity" means an increase in the duration of visible suds in a washing process cleaning soiled articles using the composition comprising a BslA-like protein, compared with the suds longevity provided by the same composition and process in the absence of the a BslA-like protein.

As used herein, the term "laundry detergent" means a liquid or solid composition, and includes, unless otherwise indicated, granular or powder-form all-purpose or "heavy-duty" washing agents, especially cleaning detergents as well as cleaning auxiliaries such as bleach additives or pre-treat types.

As used herein, the term "soiled surfaces" refers non-specifically to any type of flexible material consisting of a network of natural or artificial fibers, including natural, artificial, and synthetic fibers, such as, but not limited to, cotton, linen, wool, polyester, nylon, silk, acrylic, and the like, as well as various blends and combinations. Soiled surfaces may further refer to any type of hard surface, including natural, artificial, or synthetic surfaces, such as, but not limited to, tile, granite, grout, glass, composite, vinyl, hardwood, metal, cooking surfaces, plastic, and the like, as well as blends and combinations, as well as dishware.

As used herein, the term "water hardness" or "hardness" means uncomplexed cations ion (*i.e.,* $Ca^{2+}$ or $Mg^{2+}$) present in water that have the potential to precipitate under alkaline conditions, and thereby diminishing the surfactancy

and cleaning capacity of surfactants. Further, the terms "high water hardness" and "elevated water hardness" can be used interchangeably and are relative terms for the purposes of the present invention, and are intended to include, but not limited to, a hardness level containing at least 12 grams of calcium ion per gallon water (gpg, "American grain hardness" units).

The BslA protein

The detergent composition in accordance with the present invention comprises a BslA protein. BslA proteins have been referred to in the art as "bacterial hydrophobins", However, BslA proteins are bacterial proteins with very little sequence or structural similarity to hydrophobins, and are therefore very different proteins to hydrophobins.

BslA proteins exhibit structural and functional similarity to BslA, a protein identified in *Bacillus subtilis,* and has previously been referred to in the literature as YuaB. The wild-type BslA (WT-BslA) protein endogenous to *Bacillus subtilis* adopts a first conformation that is soluble in water, which transitions to a second conformation when adsorbed at an interface to expose hydrophobic residues to form a "hydrophobic cap". The hydrophobic cap anchors the BslA protein at the interface by extending into the non-aqueous or non-polar phase. In addition, the BslA protein in the second configuration self-assembles to form a highly structured two dimensional lattice at the interface. This two dimensional lattice forms a viscoelastic film at the interface increases the stability of the interface, and resists rearrangement or relaxation of the interface after compression or deformation. Certain variants of the BslA parent, such as the L77K mutant, do not retain the same ability as wild-type BslA to form the highly structured two dimensional lattice at the interface, presumably as the mutation destabilises the hydrophobic cap; it has significant interfacial activity, but does not form the same large-scale 2D lattice as observed with the wild-type BslA protein in which the hydrophobic cap is unaltered.

Without wishing to be bound by theory, it is suggested that BslA may form dimers and higher oligomers in the aqueous phase, via covalent bonds, such as between cysteine residues of neighboring BslA molecules, or via hydrogen bonding, for example. The formation of these BslA dimers and/or higher oligomers may slow the kinetics of adsorption via a decreased diffusion coefficient and may effectively lower the concentration of the BslA available to adsorb at an interface as only one end of a BslA dimer or oligomer can adsorb to the interface.

By the term "BslA protein" we refer to the wild-type and variants of biofilm-surface layer protein A (BslA) having at least 50%, preferably at least 60%, more preferably at least 70%, more preferably at least 80%, more preferably at least 90% and especially at least 98% identity to one or more of the following wild-type proteins: Bacillus subtilis BslA (SEQ ID NO:1), Bacillus licheniformis BslA (SEQ ID NO:2), Bacillus amyloliquefaciens BslA (SEQ ID NO:3), Bacillus pumilus BslA (SEQ ID NO:4), Bacillus subtilis Ywe A (SEQ ID NO:5).

"Variants" of a BslA protein, as used herein, includes a sequence resulting when a wild-type protein is modified by, or at, one or more amino acids (for example 1, 2, 5 or 10 amino acids). The invention includes variants in the form of truncated forms derived from a wild type BslA protein, such as a protein having the sequence of SEQ ID NO:6. SEQ ID NO:6 corresponds to the sequence of full length wild type *Bacillus subtilis* BslA, but with the N-terminal signal sequence (amino acids 1 to 28) and 13 amino acids of the N-terminal region of mature BslA removed; truncated $BslA_{42-181}$ retains wild type properties in terms of its ability to adsorb at an interface and to stabilise that interface, and thus removal of the signal sequence and extreme N-terminal 13 amino acids of the mature protein does not appear to be in any way deleterious.

It is important that variants of BslA retain the ability of the wild type protein to adsorb at an interface and to stabilise that interface. Some performance drop in a given property of variants may of course be tolerated, but the variants should retain suitable properties for the relevant application for which they are intended. Screening of variants of one of the wild-types of SEQ ID NO: 1, SEQ ID NO: 2, SEQ ID NO: 3, SEQ ID NO: 4, or SEQ ID NO: 5 can be used to identify whether they retain appropriate properties.

The variant may have "conservative" substitutions, wherein a substituted amino acid has similar structural or chemical properties to the amino acid that replaces it, for example, replacement of leucine with isoleucine. A variant may have "non-conservative" changes, for example, replacement of a glycine with a tryptophan. Variants may also include sequences with amino acid deletions or insertions, or both. Guidance in determining which amino acid residues may be substituted, inserted, or deleted without abolishing the activity of the protein may be found using computer programs well known in the art.

In one example, one conservative substitution is included in the peptide, such as a conservative substitution in SEQ ID NO:1, SEQ ID NO:2, SEQ ID NO:3, SEQ ID NO:4, or SEQ ID NO:5. In another example, 10 or fewer conservative substitutions are included in the peptide, such as five or fewer. A peptide or protein of the invention may therefore include 1, 2, 3, 4, 5, 6, 7, 8, 9, 10 or more conservative substitutions. A peptide can be produced to contain one or more conservative substitutions by manipulating the nucleotide sequence that encodes that peptide using, for example, standard procedures such as site-directed mutagenesis or PCR. Alternatively, a peptide can be produced to contain one or more conservative substitutions by using peptide synthesis methods, for example, as known in the art.

Examples of amino acids which may be substituted for an original amino acid in a protein and which are regarded as conservative substitutions include: Ser for Ala; Lys for Arg; Gln or His for Asn; Glu for Asp; Asn for Gln; Asp for Glu; Pro for Gly; Asn or Gln for His; Leu or Val for Ile; Ile or Val for Leu; Arg or Gln for Lys; Leu or Ile for Met; Met, Leu or Tyr for Phe; Thr for Ser; Ser for Thr; Tyr for Trp; Trp or Phe for Tyr; and Ile or Leu for Val.

A variant includes a "modified protein" or "mutated protein" which encompasses proteins having at least one substitution, insertion, and/or deletion of an amino acid. A modified or mutated protein may have 1, 2, 3, 4, 5, 6, 7, 8, 9 or 10 or more amino acid modifications (selected from substitutions, insertions, deletions and combinations thereof).

In one embodiment the BslA protein may comprise a variant of SEQ ID NO:1, wherein the two cysteine residues at positions 178 and 180 are substituted with non-cysteine residues. The cysteine residues at positions 178 and 180 of the WT-BslA allow the protein to form multimers (i.e. dimers, tetramers, hexamers and potentially higher order oligomers) in solution due to the formation of disulfide bonds between the cysteine residues of adjacent WT-BslA monomers. These multimers are also surface active, if to a lesser extent than monomeric BslA.

The introduction of a reducing agent, such as 2-mercaptoethanol or dithiothreitol, for example, increases the surface activity of the BslA protein, observed in a reduction in the surface tension of the interface. Without wishing to be bound by theory, it is suggested that the reducing agent reduces the cysteine groups, thereby preventing the formation of disulfide bonds between individual BslA proteins, such that the BslA protein is monomeric in solution. Accordingly, the reduction of the cysteine groups within the B. subtilis wild-type BslA of SEQ ID NO:1 with a reducing agent improves the surfactant properties of the protein. However, such reducing agents are not suitable for many applications. Accordingly, the provision of a modified BslA where the cysteine residues have been substituted with non-cysteine residues ensures that there is no possibility of disulfide bonds forming between BslA protein monomers due to the lack of sulfur atoms within the protein. Accordingly, the resultant mutant BslA protein provides increased surface activity over its wild-type parent without requiring the application of reducing agents. The cysteine residues may be substituted for any other amino acid that does not comprise a sulfur atom, and the modified BslA protein of Bacillus subtilis BslA (SEQ ID NO:1) may correspond to SEQ ID NO: 7. For example, the substitution may be to replace the cysteine residues with alanine residues (C178A/C180A), valine residues (C178V/C180V), leucine residues (C178L/C180L) or isoleucine residues (C178I/C180I). Suitably, the substitution does not affect the folding of the protein. Typically, the conformation of the modified BslA protein is similar to the wild-type BslA monomer. Preferably, the conformation of the modified protein is substantially the same as the wild-type BslA monomer in solution. Preferably, the cysteine residues are substituted with alanine residues, for example the BslA protein of SEQ ID NO:8 which is a variant of Bacillus subtilis BslA (SEQ ID NO:1).

The invention also covers any fragment of SEQ ID NO:1, SEQ ID NO:2, SEQ ID NO:3, SEQ ID NO:4, or SEQ ID NO:5 that can adsorb to an interface and stabilise that interface. According to the invention, the term "fragment" is intended to mean an amino acid sequence of at least 30, 60, 100, 150 contiguous amino acids of the reference sequences or any integer there between. For example, the invention includes truncated forms of the wild type BslA (e.g. $BslA_{42-181}$, SEQ ID NO:6).

Peptides can be modified by a variety of chemical techniques to produce derivatives having essentially the same activity as the unmodified peptides, and optionally having other desirable properties. For example, carboxylic acid groups of the protein, whether carboxyl-terminal or side chain, may be provided in the form of a salt of a pharmaceutically-acceptable cation or esterified, for example to form a C1-C6 alkyl ester, or converted to an amide, for example of formula $CONR_1R_2$ wherein $R_1$ and $R_2$ are each independently H or C1-C6 alkyl, or combined to form a heterocyclic ring, such as a 5- or 6-membered ring. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be modified to C1-C6 alkyl or dialkyl amino or further converted to an amide. Hydroxyl groups of the peptide side chains may be converted to alkoxy or ester groups, for example C1-C6 alkoxy or C1-C6 alkyl ester, using well-recognized techniques. Phenyl and phenolic rings of the peptide side chains may be substituted with one or more halogen atoms, such as F, Cl, Br or I, or with C1-C6 alkyl, C1-C6 alkoxy, carboxylic acids and esters thereof, or amides of such carboxylic acids. Methylene groups of the peptide side chains can be extended to homologous C2-C4 alkylenes. Thiols can be protected with any one of a number of well-recognized protecting groups, such as acetamide groups. Those skilled in the art will also recognize methods for introducing cyclic structures into the peptides of this disclosure to select and provide conformational constraints to the structure that result in enhanced stability.

The sequence of the BslA protein according to the present invention is preferably at least 50% identical to SEQ ID NO:1, SEQ ID NO:2, SEQ ID NO:3, SEQ ID NO:4, or SEQ ID NO:5, more preferably at least 60% identical, yet more preferably at least 70% identical, at least 75% identical, at least 80% identical, at least 90% identical, at least 95% identical, or even at least 99% identical. For the purpose of the present invention, these variant BslA proteins possessing this high level of identity to wild-type BslA are embraced within the term "BslA protein".

The term "sequence identity" refers to the identity between two or more amino acid sequences and is expressed in terms of the identity or similarity between the sequences. Sequence identity can be measured in terms of percentage identity; the higher the percentage, the more identical the sequences are. The percentage identity is calculated over the length of comparison, e.g. in the present invention it is typically calculated over the entire length of a sequence aligned

against the entire length of SEQ ID NO:1, SEQ ID NO:2, SEQ ID NO:3, SEQ ID NO:4, or SEQ ID NO:5.

Methods of alignment of sequences for comparison are well known in the art and identity can be calculated by many known methods. Various programs and alignment algorithms are described in the art.2 10 It should be noted that the terms 'sequence identity' and 'sequence similarity' are often used inconsistently and interchangeably in the art.

Identity, or homology, percentages as mentioned herein in respect of the present invention are those that can be calculated with the GAP program, obtainable from GCG (Genetics Computer Group Inc., Madison, WI, USA). Alternatively, a manual alignment can be performed.

For polypeptide sequence comparison the following settings can be used:

Alignment algorithm: Needleman and Wunsch, J. Mol. Biol. 1970, 48: 443-453.
As a comparison matrix for amino acid similarity the Blosum62 matrix is used (Henikoff S. and Henikoff J.G., P.N.A.S. USA 1992, 89: 10915-10919).

The following gap scoring parameters are used:

Gap penalty: 12
Gap length penalty: 2
No penalty for end gaps.

A given sequence is typically compared against the full-length sequence of SEQ ID NO: 1, SEQ ID NO: 2, SEQ ID NO: 3, SEQ ID NO: 4, or SEQ ID NO: 5 to obtain a score.

The composition of the invention comprises from about 0.001 to about 5%, preferably from about 0.005 to about 2%, more preferably from about 0.01 to about 1% most preferably from about 0.03 to about 0.5% by weight of the composition of BslA protein based on active protein.

Surfactant system

The detergent comprises from 1% to 60% more preferably from about 5% to about 50% by weight thereof of a surfactant system. The surfactant system comprises surfactants selected from the group consisting of anionic surfactants, cationic surfactants, nonionic surfactants, amphoteric surfactants, zwitterionic surfactants, and mixtures thereof. Preferably, the surfactants comprise an anionic surfactant selected from the group consisting of alkyl benzene sulfonate, alkoxylated alkyl sulfates, alkyl sulfates, and mixtures thereof.

The surfactant system for providing food cleaning and good suds profile comprises i) an anionic surfactant; and ii) an amphoteric and/or zwitterionic surfactant. The weight ratio of anionic surfactant to amphoteric and/or zwitterionic surfactant is less than 9:1, preferably less than 5:1 to about 1:2, more preferably from about 4:1 to about 1:1 and especially from about 4:1 to about 2:1

Extremely useful surfactant systems for use herein include those comprising anionic surfactants and comprising in addition, amine oxide and/or betaine surfactants. Amine oxide surfactants are particularly preferred. Preferably the surfactant system comprises an anionic surfactant selected from alkyl sulphate, alkyl alkoxy sulphate especially alkyl ethoxy sulphate, and mixtures thereof, in combination with amine oxide, most preferably in a weight % ratio of less than 9:1, more preferably less than 5:1 to about 1:2, more preferably from about 4:1 to about 1:1 and especially from about 4:1 to about 2:1.

Another preferred surfactant system for use herein comprises an anionic and amphoteric/zwitterionic system in which the amphoteric to zwitterionic weight ratio is preferably from about 2:1 to about 1:2. In particular a system in which the amphoteric surfactant comprises an amine oxide surfactant and the zwitteronic surfactant comprises a betaine. Preferred ratios of amine oxide to betaine are from 1.5:1 to 1:1.5, preferably from 1.2:1 to 1:1.2, most preferably about 1:1.

Also preferred for use herein are surfactant systems comprising non-ionic surfactants. Especially preferred surfactant systems for the composition of the invention comprise an anionic surfactant preferably selected from the group consisting of alkyl sulphate, alkyl alkoxy sulphate and mixtures thereof, more preferably an alkoxylated sulfate. Preferred surfactant systems comprise in addition an amphoteric surfactant, preferably an amine oxide surfactant. Preferred surfactant systems comprise a non-ionic surfactant. In summary, the most preferred surfactant system for use herein comprises an alkoxylated sulfate surfactant, amine oxide and non-ionic surfactant. Most preferably the nonionic surfactant is an alkoxylated alcohol surfactant, especially an ethoxylated alcohol surfactant.

Anionic surfactant

Anionic surfactants include, but are not limited to, those surface-active compounds that contain an organic hydrophobic group containing generally 8 to 22 carbon atoms or generally 8 to 18 carbon atoms in their molecular

structure and at least one water-solubilizing group preferably selected from sulfonate, sulfate, and carboxylate so as to form a water-soluble compound. Usually, the hydrophobic group will comprise a C 8-C 22 alkyl, and/or acyl group. Such surfactants are employed in the form of water-soluble salts and the salt-forming cation usually is selected from sodium, potassium, ammonium, magnesium and mono-, di- or tri-C 2-C 3 alkanolammonium, with the sodium, cation being the usual one chosen.

The surfactant system comprises an anionic surfactant or mixtures thereof. The anionic surfactant comprises any anionic cleaning surfactant, preferably selected from anionic sulphate or sulphonate surfactants or mixtures thereof.

Preferably the anionic surfactant is an alkoxylated alkyl sulphate surfactant, preferably an ethoxylated alkyl sulphate surfactant, preferably having an average ethoxylation degree of from about 0.2 to about 3, more preferably from about 0.3 to about 2, even more preferably from about 0.4 to about 1.5, and especially from about 0.4 to about 1. When the anionic surfactant is a mixture of surfactants, the alkoxylation degree is the weight average alkoxylation degree of all the components of the mixture (weight average alkoxylation degree). In the weight average alkoxylation degree calculation the weight of anionic surfactant components not having alkoxylated groups should also be included.
Weight average alkoxylation degree = (x1 * alkoxylation degree of surfactant 1 + x2 * alkoxylation degree of surfactant 2 + ....) / (x1 + x2 + ....) wherein x1, x2, ... are the weights in grams of each anionic surfactant of the mixture and alkoxylation degree is the number of alkoxy groups in each anionic surfactant.

Also preferred are branched anionic surfactants, typically having a weight average level of branching of from 2 to 60% by weight, particularly those having a weight average level of branching of from about 5% to about 40%.

Preferably the anionic surfactant to be used in the detergent of the present invention comprises a branched anionic surfactant having a level of branching of from about 5% to about 40%, preferably from about 10 to about 35% and more preferably from about 20% to about 30%. Preferably, the branching group is an alkyl. Typically, the alkyl is selected from methyl, ethyl, propyl, butyl, pentyl, cyclic alkyl groups and mixtures thereof. Single or multiple alkyl branches could be present on the main hydrocarbyl chain of the starting alcohol(s) used to produce the anionic surfactant used in the detergent of the invention. Most preferably the branched anionic surfactant is selected from alkyl sulphates, alkyl ethoxy sulphates, and mixtures thereof.

The branched anionic surfactant can be a single anionic surfactant or a mixture of anionic surfactants. In the case of a single surfactant the percentage of branching refers to the weight percentage of the hydrocarbyl chains that are branched in the original alcohol from which the surfactant is derived.

In the case of a surfactant mixture the percentage of branching is the weight average and it is defined according to the following formula:

$$\text{Weight average of branching (\%)} = [(x1 * \text{wt\% branched alcohol 1 in alcohol 1} + x2 * \text{wt\% branched alcohol 2 in alcohol 2} + \ldots) / (x1 + x2 + \ldots)] * 100$$

wherein x1, x2, ... are the weight in grams of each alcohol in the total alcohol mixture of the alcohols which were used as starting material for the anionic surfactant for the detergent of the invention. In the weight average branching degree calculation the weight of anionic surfactant components not having branched groups should also be included.

It may be preferred that the surfactant system comprises at least 50%, more preferably at least 60% and preferably at least 70% of branched anionic surfactant by weight of the surfactant system. In a particularly preferred surfactant system, the branched anionic surfactant comprises more than 50% by weight thereof of an alkyl ethoxylated sulphate having an ethoxylation degree of from about 0.1 to 5 or 0.2 to about 3 and preferably a level of branching of from about 5% to about 40%.

Preferably, the branched anionic surfactant comprises at least 50%, more preferably at least 60% and especially at least 70% of a sulphate surfactant by weight of the branched anionic surfactant. Especially preferred detergents from a cleaning view point art those in which the branched anionic surfactant comprises more than 50%, more preferably at least 60% and especially at least 70% by weight thereof of sulphate surfactant and the sulphate surfactant is selected from the group consisting of alkyl sulphate, alkyl ethoxy sulphates and mixtures thereof. Even more preferred are those in which the branched anionic surfactant has a degree of ethoxylation of from about 0.2 to about 3, more preferably from about 0.3 to about 2, even more preferably from about 0.4 to about 1.5, and especially from about 0.4 to about 1 and even more preferably when the anionic surfactant has a level of branching of from about 10% to about 35%, more preferably from about 20% to 30%.

Sulphate Surfactants

Preferably the surfactant comprises anionic sulphate surfactants. Anionic sulphate surfactants selected from the group consisting of alkyl sulphate, alkyl alkoxy sulphate and mixtures thereof may be particularly preferred, especially

for dishwashing compositions.

Especially preferred are alkoxylated anionic surfactants, more preferably alkyl alkoxy sulphate surfactant. Preferred alkyl alkoxyl sulphates for use herein are alkyl ethoxy sulphates. Suitable sulphate surfactants for use herein include water-soluble salts of C8-C18 alkyl or hydroxyalkyl, sulphate and/or ether sulfate. Suitable counterions include alkali metal cation or ammonium or substituted ammonium, but preferably sodium.

The sulphate surfactants may be selected from C8-C18 primary, branched chain and random alkyl sulphates (AS); C8-C18 secondary (2,3) alkyl sulphates; C8-C18 alkyl alkoxy sulphates (AExS) wherein preferably x is from 1-30 in which the alkoxy group could be selected from ethoxy, propoxy, butoxy or even higher alkoxy groups and mixtures thereof. The alkoxylated anionic surfactant typically has an average alkoxylation degree of from about 0.1 to 11 or 0.1 to 7, preferably from about 0.2 to about 4, even more preferably from about 0.3 to about 3, even more preferably from about 0.4 to about 1.5 and especially from about 0.4 or 0.2 to about 1. Preferably, the alkoxy group is ethoxy.

Alkyl sulfates and alkyl alkoxy sulfates are commercially available with a variety of chain lengths, ethoxylation and branching degrees. Commercially available sulphates include, those based on Neodol alcohols ex the Shell company, Lial - Isalchem and Safol ex the Sasol company, natural alcohols ex The Procter & Gamble Chemicals company.

Preferably, the surfactant system comprises alkyl sulfates and/or alkyl ethoxy sulfates; more preferably a combination of alkyl sulfates and/or alkyl ethoxy sulfates with a combined average ethoxylation degree of less than 5, preferably less than 3, more preferably less than 2 and more than 0.5. Preferably the anionic surfactant has an average level of branching of from about 5% to about 40%.

Sulphonate Surfactants

Suitable sulphonate surfactants for use herein include water-soluble salts of C8-C18 alkyl or hydroxyalkyl sulphonates; C11-C18 alkyl benzene sulphonates (LAS), modified alkylbenzene sulphonate (MLAS) as discussed in WO 99/05243, WO 99/05242, WO 99/05244, WO 99/05082, WO 99/05084, WO 99/05241, WO 99/07656, WO 00/23549, and WO 00/23548; methyl ester sulphonate (MES); and alpha-olefin sulphonate (AOS). Those also include the paraffin sulphonates may be monosulphonates and/or disulphonates, obtained by sulphonating paraffins of 10 to 20 carbon atoms. The sulfonate surfactant also includes the alkyl glyceryl sulphonate surfactants. In particular, for a laundry detergent the anionic surfactant preferably comprises at least 40% or more preferably at least 50% or at least 60% or even at least 80 or 90% sulphonate surfactant.

Fatty acids

Water-soluble salts of the higher fatty acids, i.e., "soaps", may also be useful anionic surfactants in the cleaning compositions of the present invention, particularly for laundry detergents. This includes alkali metal soaps such as the sodium, potassium, ammonium, and alkyl ammonium salts of higher fatty acids containing from about 8 to about 24 carbon atoms, and preferably from about 12 to about 18 carbon atoms. Soaps can be made by direct saponification of fats and oils or by the neutralization of free fatty acids. Such alkali salts include monovalent or divalent alkali metal salts like sodium, potassium, lithium and/or magnesium salts as well as the ammonium and/or alkylammonium salts of fatty acids, preferably the sodium salt. Particularly useful are the sodium and potassium salts of the mixtures of fatty acids derived from coconut oil, palm kernel oil and tallow, i.e., sodium or potassium tallow, palm kernel and coconut soap. The detergent composition may comprise from about 0.1 wt% to about 10 wt%, preferably from about 0.5 wt% to about 3 wt%, more preferably from about 1 wt% to about 1.5 wt%, of one or more fatty acids and/or alkali salts thereof. This may be particularly advantageous to provide improved rinsing. However, the cleaning compositions of the present invention preferably contains soaps at a relatively low level, e.g., no more than about 5 wt%, more preferably not more than about 2 wt% or 1 wt%, and most preferably said cleaning composition is essentially free of soaps. Where fatty acids are added, they preferably contain very low levels of unsaturated fatty acids, particularly oleic acid. Levels of oleic acid in the composition are preferably below 0.5, more preferably below 0.3, more preferably below 0.2 or even below 0.1 wt% of the compositions, most preferably essentially free of oleic acid. Higher levels may be accommodated however, additional enzyme may need to be present to counteract the competition caused by their presence. Where oleic acid is incorporated, it may be preferred to also incorporate enzyme stabilizer. Physical stabilization such as by encapsulation may be particularly preferred.

Non-ionic surfactant

Nonionic surfactant, when present, is typically present in an amount of from 0.05% to 30%, preferably 0.1% to 20%, most preferably 0.5% to 10% or 0.5% to 7% or even 0.5% to 3% by weight of the composition. The nonionic surfactant is preferably present in the surfactant system in amounts from 1 to 60 wt% of the surfactant system, and particularly for laundry detergents preferably from 2 to 60, or 5 to 55 wt% based on the surfactant system. Suitable

nonionic surfactants include the condensation products of aliphatic alcohols with from 1 to 25 moles of ethylene oxide. The alkyl chain of the aliphatic alcohol can either be straight or branched, primary or secondary, and generally contains from 8 to 22 carbon atoms. Particularly preferred are the condensation products of alcohols having an alkyl group containing from 10 to 18 carbon atoms, preferably from 10 to 15 carbon atoms with from 2 to 18 moles, preferably 2 to 15, more preferably 5-12 of ethylene oxide per mole of alcohol. Highly preferred nonionic surfactants are the condensation products of Guerbet alcohols with from 2 to 18 moles, preferably 2 to 15, more preferably 5-12 of ethylene oxide per mole of alcohol.

However, in certain preferred embodiments of the present invention, particularly for dishwashing the cleaning composition contains nonionic surfactants at a relatively low level, e.g., no more than about 3 wt%, more preferably not more than about 2 wt% or 1 wt%, and most preferably said cleaning composition is essentially free of nonionic surfactants.

Other surfactants useful herein include amphoteric surfactants, zwitterionic surfactants and cationic surfactants. Such surfactants are typically present at levels from about 0.2 wt%, 0.5 wt% or 1 wt% to about 10 wt%, 20 wt% or 30 wt%. Preferably, the composition of the present invention will further comprise amphoteric and/or zwitterionic surfactant, more preferably an amine oxide and/or betaine surfactant, most preferably an amine oxide.

In a preferred but not necessary embodiment of the present invention, the cleaning composition is a liquid dish detergent composition containing from about 0.5 wt% to about 20 wt% of one or more amphoteric and/or zwitterionic surfactants, preferably amine oxide.

Amphoteric surfactant

Preferred amphoteric surfactants are selected from the group consisting of amine oxide surfactants, such as, for example, alkyl dimethyl amine oxide or alkyl amido propyl dimethyl amine oxide, more preferably alkyl dimethyl amine oxide and especially coco dimethyl amino oxide. Amine oxide may have a linear or mid-branched alkyl moiety. Typical linear amine oxides include water-soluble amine oxides containing one R1 C8-18 alkyl moiety and 2 R2 and R3 moieties selected from the group consisting of C1-3 alkyl groups and C1-3 hydroxyalkyl groups. Preferably amine oxide is characterized by the formula R1 - N(R2)(R3) O wherein R1 is a C8-18 alkyl and R2 and R3 are selected from the group consisting of methyl, ethyl, propyl, isopropyl, 2-hydroxethyl, 2-hydroxypropyl and 3-hydroxypropyl. The linear amine oxide surfactants in particular may include linear C10-C18 alkyl dimethyl amine oxides and linear C8-C12 alkoxy ethyl dihydroxy ethyl amine oxides. Preferred amine oxides include linear C10, linear C10-C12, and linear C12-C14 alkyl dimethyl amine oxides. As used herein "mid-branched" means that the amine oxide has one alkyl moiety having n1 carbon atoms with one alkyl branch on the alkyl moiety having n2 carbon atoms. The alkyl branch is located on the $\alpha$ carbon from the nitrogen on the alkyl moiety. This type of branching for the amine oxide is also known in the art as an internal amine oxide. The total sum of n1 and n2 is from 10 to 24 carbon atoms, preferably from 12 to 20, and more preferably from 10 to 16. The number of carbon atoms for the one alkyl moiety (n1) should be approximately the same number of carbon atoms as the one alkyl branch (n2) such that the one alkyl moiety and the one alkyl branch are symmetric. As used herein "symmetric" means that |n1 - n2| is less than or equal to 5, preferably 4, most preferably from 0 to 4 carbon atoms in at least 50 wt%, more preferably at least 75 wt% to 100 wt% of the mid-branched amine oxides for use herein. The amine oxide further comprises two moieties independently selected from a C1-3 alkyl, a C1-3 hydroxyalkyl group, or a polyethylene oxide group containing an average of from about 1 to about 3 ethylene oxide groups. Preferably the two moieties are selected from a C1-3 alkyl, more preferably both are selected as a C1 alkyl. Most preferably the amine oxide is alkyl dimethyl amine oxide, especially C10-C18 alkyl dimethyl amine oxide.

Zwitterionic surfactant

Other suitable surfactants include betaines, such as alkyl betaines, alkylamidobetaines, amidazoliniumbetaines, sulfobetaines (also referred to as INCI sultaines) as well as the phosphobetaines. Preferred betaines meet formula I:

$$R^1\text{-}[CO\text{-}X(CH_2)_n]_x\text{-}N^+(R^2)(R_3)\text{-}(CH_2)_m\text{-}[CH(OH)\text{-}CH_2]_y\text{-}Y^- \qquad (I)$$

wherein

$R^1$ is a saturated or unsaturated C6-22 alkyl residue, preferably C8-18 alkyl residue, in particular a saturated C10-16 alkyl residue, for example a saturated C12-14 alkyl residue;
X is NH, $NR^4$ with C1-4 Alkyl residue $R^4$, O or S,
n a number from 1 to 10, preferably 2 to 5, in particular 3,
x 0 or 1, preferably 1,
$R^2$, $R^3$ are independently a C1-4 alkyl residue, potentially hydroxy substituted such as a hydroxyethyl, preferably a methyl.

m a number from 1 to 4, in particular 1, 2 or 3,
y 0 or 1 and
Y is COO, SO3, OPO($OR^5$)O or P(O)($OR^5$)O, whereby $R^5$ is a hydrogen atom H or a C1-4 alkyl residue.

Preferred betaines are the alkyl betaines of the formula (Ia), the alkyl amido propyl betaine of the formula (Ib), the sulfo betaines of the formula (Ic) and the amido sulfobetaines of the formula (Id);

$R^1\text{-}N^+(CH_3)_2\text{-}CH_2COO^-$ (Ia)

$R^1\text{-}CO\text{-}NH(CH_2)_3\text{-}N^+(CH_3)_2\text{-}CH_2COO^-$ (Ib)

$R^1\text{-}N^+(CH_3)_2\text{-}CH_2CH(OH)CH_2SO_3\text{-}$ (Ic)

$R^1\text{-}CO\text{-}NH\text{-}(CH_2)_3\text{-}N^+(CH_3)_2\text{-}CH_2CH(OH)CH_2SO_3\text{-}$ (Id)

in which $R^1$1 as the same meaning as in formula I. Particularly preferred betaines are the carbobetaines [wherein $Y^-$ $=COO^-$], in particular the carbobetaines of the formula (Ia) and (Ib), more preferred are the alkylamidobetaines of the formula (Ib).

Examples of suitable betaines and sulfobetaines are the following [designated in accordance with INCI]: almondamidopropyl of betaines, apricotam idopropyl betaines, avocadamidopropyl of betaines, babassuamidopropyl of betaines, behenam idopropyl betaines, behenyl of betaines, betaines, canolam idopropyl betaines, capryl/capram idopropyl betaines, carnitine, cetyl of betaines, cocamidoethyl of betaines, cocam idopropyl betaines, cocam idopropyl hydroxysultaine, coco betaines, coco hydroxysultaine, coco/oleam idopropyl betaines, coco sultaine, decyl of betaines, dihydroxyethyl oleyl glycinate, dihydroxyethyl soy glycinate, dihydroxyethyl stearyl glycinate, dihydroxyethyl tallow glycinate, dimethicone propyl of PG-betaines, erucam idopropyl hydroxysultaine, hydrogenated tallow of betaines, isostearam idopropyl betaines, lauram idopropyl betaines, lauryl of betaines, lauryl hydroxysultaine, lauryl sultaine, milkam idopropyl betaines, minkamidopropyl of betaines, myristam idopropyl betaines, myristyl of betaines, oleam idopropyl betaines, oleam idopropyl hydroxysultaine, oleyl of betaines, olivamidopropyl of betaines, palmam idopropyl betaines, palm itam idopropyl betaines, palmitoyl Carnitine, palm kernelam idopropyl betaines, polytetrafluoroethylene acetoxypropyl of betaines, ricinoleam idopropyl betaines, sesam idopropyl betaines, soyam idopropyl betaines, stearam idopropyl betaines, stearyl of betaines, tallowam idopropyl betaines, tallowam idopropyl hydroxysultaine, tallow of betaines, tallow dihydroxyethyl of betaines, undecylenam idopropyl betaines and wheat germam idopropyl betaines. A preferred betaine is, for example, cocoamidopropylbetaine.

The most preferred surfactant system particularly for a dishwashing detergent composition of the present invention comprises: (i) 1% to 40%, preferably 6% to 32%, more preferably 8% to 25% weight of the total composition of an anionic surfactant, preferably comprising an alkoxylated sulfate surfactant (ii) 0.01% to 20%wt, preferably from 0.2% to 15%wt, more preferably from 0.5% to 10% by weight of the composition of amphoteric and/or zwitterionic and/or nonionic surfactant. Preferred compositions comprise 0.01% to 20 wt% of the composition of amphoteric and nonionic surfactant, most preferably wherein the amphoteric surfactant comprises amine oxide surfactant. It has been found that such surfactant system in combination with the BslA protein will provide the excellent cleaning required from a manual dishwashing detergent while having very good suds profile, especially in the presence of greasy soils and break-down products of greasy soils, and provides a good finish of the washed items.

Enzymes

Suitable enzymes include protease such as metalloprotease or alkaline serine protease, such as subtilisin, amylase, lipase, cellulase, mannanase, pectinase, DNAse, oxidoreductase, peroxidases, lipases, phospholipases, cellobiohydrolases, cellobiose dehydrogenases, esterases, cutinases, pectinases, pectate lyases, keratinases, reductases, oxidases, phenoloxidases, lipoxygenases, ligninases, pullulanases, tannases, pentosanases, glucanases, arabinosidases, hyaluronidase, chondroitinase, laccases, amylases, and mixtures thereof.

Preferred compositions of the invention comprise one or more enzymes selected from lipases, proteases, cellulases, amylases and any combination thereof.

Each additional enzyme is typically present in an amount from0.0001 to 1 wt% (weight of active protein) more preferably from 0.0005 to 0.5 wt%, most preferably 0.005-0.1%). It may be particularly preferred for the compositions of the present invention to additionally comprise a lipase enzyme. Suitable lipases include those of bacterial, fungal or synthetic origin, and variants thereof. Chemically modified or protein engineered mutants are also suitable. Examples of suitable lipases include lipases from *Humicola* (synonym *Thermomyces*), e.g., from *H. lanuginosa* (*T. lanuginosus*).

The lipase may be a "first cycle lipase", e.g. such as those described in WO06/090335 and WO13/116261. In

one aspect, the lipase is a first-wash lipase, preferably a variant of the wild-type lipase from Thermomyces lanuginosus comprising T231R and/or N233R mutations. Preferred lipases include those sold under the tradenames Lipex®, Lipolex® and Lipoclean® by Novozymes, Bagsvaerd, Denmark.

Other suitable lipases include: Liprl 139, e.g. as described in WO2013/171241; and TfuLip2, e.g. as described in WO2011/084412 and WO2013/033318.

It may be particularly preferred for the compositions of the present invention to additionally comprise a protease enzyme. Suitable proteases include metalloproteases and/or serine proteases. Examples of suitable neutral or alkaline proteases include: subtilisins (EC 3.4.21.62); trypsin-type or chymotrypsin-type proteases; and metalloproteases. The suitable proteases include chemically or genetically modified mutants of the aforementioned suitable proteases.

Suitable commercially available protease enzymes include those sold under the trade names Alcalase®, Savinase®, Primase®, Durazym®, Polarzyme®, Kannase®, Liquanase®, Liquanase Ultra®, Savinase Ultra®, Ovozyme®, Neutrase®, Everlase® and Esperase® by Novozymes A/S (Denmark), those sold under the tradename Maxatase®, Maxacal®, Maxapem®, Preferenz P® series of proteases including Preferenz® P280, Preferenz® P281, Preferenz® P2018-C, Preferenz® P2081-WE, Preferenz® P2082-EE and Preferenz® P2083-A/J, Properase®, Purafect®, Purafect Prime®, Purafect Ox®, FN3®, FN4®, Excellase® and Purafect OXP® by DuPont, those sold under the tradename Opticlean® and Optimase® by Solvay Enzymes, those available from Henkel/ Kemira, namely BLAP (sequence shown in Figure 29 of US 5,352,604 with the folowing mutations S99D + S101 R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D) - all from Henkel/Kemira; and KAP (Bacillus alkalophilus subtilisin with mutations A230V + S256G + S259N) from Kao.

A suitable protease is described in WO11/140316 and WO11/072117.

It may be particularly preferred for the compositions of the present invention to additionally comprise an amylase enzyme. Preferred amylases are derived from AA560 alpha amylase endogenous to Bacillus sp. DSM 12649, preferably having the following mutations: R118K, D183*, G184*, N195F, R320K, and/or R458K. Suitable commercially available amylases include Stainzyme®, Stainzyme® Plus, Natalase, Termamyl®, Termamyl® Ultra, Liquezyme® SZ, Duramyl®, Everest® (all Novozymes) and Spezyme® AA, Preferenz S® series of amylases, Purastar® and Purastar® Ox Am, Optisize® HT Plus (all Du Pont). A suitable amylase is described in WO06/002643.

It may be particularly preferred for the compositions of the present invention to additionally comprise a cellulase enzyme. Suitable cellulases include those of bacterial or fungal origin. Chemically modified or protein engineered mutants are also suitable. Suitable cellulases include cellulases from the genera *Bacillus, Pseudomonas, Humicola, Fusarium, Thielavia, Acremonium,* e.g., the fungal cellulases produced from *Humicola insolens, Myceliophthora thermophila* and *Fusarium oxysporum.*

Commercially available cellulases include Celluzyme®, Carezyme®, and Carezyme® Premium, Celluclean® and Whitezyme® (Novozymes A/S), Revitalenz® series of enzymes (Du Pont), and Biotouch® series of enzymes (AB Enzymes). Suitable commercially available cellulases include Carezyme® Premium, Celluclean® Classic.

Enzyme Stabilizer

Preferably the composition of the invention comprises an enzyme stabilizer. Suitable enzyme stabilizers may be selected from the group consisting of (a) univalent, bivalent and/or trivalent cations preferably selected from the group of inorganic or organic salts of alkaline earth metals, alkali metals, aluminum, iron, copper and zinc, preferably alkali metals and alkaline earth metals, preferably alkali metal and alkaline earth metal salts with halides, sulfates, sulfites, carbonates, hydrogencarbonates, nitrates, nitrites, phosphates, formates, acetates, propionates, citrates, maleates, tartrates, succinates, oxalates, lactates, and mixtures thereof. In a preferred embodiment the salt is selected from the group consisting of sodium chloride, calcium chloride, potassium chloride, sodium sulfate, potassium sulfate, sodium acetate, potassium acetate, sodium formate, potassium formate, calcium lactate, calcium nitrate and mixtures thereof. Most preferred are salts selected from the group consisting of calcium chloride, potassium chloride, potassium sulfate, sodium acetate, potassium acetate, sodium formate, potassium formate, calcium lactate, calcium nitrate, and mixtures thereof, and in particular potassium salts selected from the group of potassium chloride, potassium sulfate, potassium acetate, potassium formate, potassium propionate, potassium lactate and mixtures thereof. Most preferred are potassium acetate and potassium chloride. Preferred calcium salts are calcium formate, calcium lactate and calcium nitrate including calcium nitrate tetrahydrate. Calcium and sodium formate salts may be preferred. These cations are present at at least about 0.01 wt%, preferably at least about 0.03 wt%, more preferably at least about 0.05 wt%, most preferably at least about 0.25 wt% up to about 2 wt% or even up to about 1 wt% by weight of the total composition. These salts are formulated from about 0.1 to about 5 wt%, preferably from about 0.2 to about 4 wt%, more preferably from about 0.3 to about 3 wt%, most preferably from about 0.5 to about 2 wt% relative to the total weight of the composition. Further enzyme stabilizers can be selected from the group (b) carbohydrates selected from the group consisting of oligosaccharides, polysaccharides and mixtures thereof, such as a monosaccharide glycerate as described in WO201219844; (c) mass

efficient reversible protease inhibitors selected from the group consisting of phenyl boronic acid and derivatives thereof, preferably 4-formyl phenylboronic acid; (d) alcohols such as 1,2-propane diol, propylene glycol; (e) peptide aldehyde stabilizers such as tripeptide aldehydes such as Cbz-Gly-Ala-Tyr-H, or disubstituted alaninamide; (f) carboxylic acids such as phenyl alkyl dicarboxylic acid as described in WO2012/19849 or multiply substituted benzyl carboxylic acid comprising a carboxyl group on at least two carbon atoms of the benzyl radical such as described in WO2012/19848, phthaloyl glutamine acid, phthaloyl asparagine acid, aminophthalic acid and/or an oligoamino-biphenyl-oligocarboxylic acid; and; (g) mixtures thereof. An example of a suitable mixture comprises: (1) reversible protease inhibitors such as a boron containing compound; (2) 1-2 propane diol; (3) calcium formate and/or sodium formate; and (4) any combination thereof.

If the cleaning composition of the present invention is provided in a powder form, it may also be especially preferred for the powder to comprise low levels, or even be essentially free, of builder. The term "essentially free" means that the composition "comprises no deliberately added" amount of that ingredient. In a preferred embodiment, the cleaning composition of the present invention comprises no builder.

Chelant

The detergent composition herein typically comprises a chelant at a level of from 0.1% to 20%, preferably from 0.2% to 5%, more preferably from 0.2% to 3% by weight of total composition.

As commonly understood in the detergent field, chelation herein means the binding or complexation of a bi- or multidentate ligand. These ligands, which are often organic compounds, are called chelants, chelators, chelating agents, and/or sequestering agent. Chelating agents form multiple bonds with a single metal ion. Chelants, are chemicals that form soluble, complex molecules with certain metal ions, inactivating the ions so that they cannot normally react with other elements or ions to produce precipitates or scale, or forming encrustations on soils turning them harder to be removed. The ligand forms a chelate complex with the substrate. The term is reserved for complexes in which the metal ion is bound to two or more atoms of the chelant. Suitable chelating agents can be selected from the group consisting of amino carboxylates, amino phosphonates, polycarboxylate chelating agents and mixtures thereof.

Preferred chelants for use herein are the amino acids based chelants and preferably glutamic-N,N- diacetic acid (GLDA), methylglycine-N,N-diacetic acid (MGDA), and derivatives, and/or phosphonate based chelants and preferably diethylenetriamine penta methylphosphonic acid or hydroxyethyldiphosphonic acid. GLDA (salts and derivatives thereof) is especially preferred according to the invention, with the tetrasodium salt thereof being especially preferred.

Other chelants include homopolymers and copolymers of polycarboxylic acids and their partially or completely neutralized salts, monomeric polycarboxylic acids and hydroxycarboxylic acids and their salts. Suitable polycarboxylic acids are acyclic, alicyclic, heterocyclic and aromatic carboxylic acids, in which case they contain at least two carboxyl groups which are in each case separated from one another by, preferably, no more than two carbon atoms. A suitable hydroxycarboxylic acid is, for example, citric acid. Another suitable polycarboxylic acid is the homopolymer of acrylic acid. Preferred are the polycarboxylates end capped with sulfonates.

Solvents

When the cleaning composition is in the form of a liquid detergent composition, particularly a laundry or liquid dishwashing detergent it may further comprise one or more organic solvents, which can be present in an amount ranging from about 1 wt% to about 80 wt%, preferably 5 wt% to about 70 wt%, more preferably from about 10wt% to about 60 wt%, even more preferably from about 15 wt% to about 50 wt%, and most preferably from about 20 wt% to about 45 wt%, by total weight of the composition. Preferably the composition comprises cleaning solvents, especially when the composition is a dishwashing composition.

Cleaning solvents

The liquid compositions of the present invention may comprise a grease cleaning solvent, or mixtures thereof as a highly preferred optional ingredient. Suitable solvent is selected from the group consisting of ethers and diethers having from 4 to 14 carbon atoms, preferably from 6 to 12 carbon atoms, and more preferably from 8 to 10 carbon atoms; glycols or alkoxylated glycols; alkoxylated aromatic alcohols; aromatic alcohols; alkoxylated aliphatic alcohols; aliphatic alcohols; C8-C14 alkyl and cycloalkyl hydrocarbons and halohydrocarbons; C6-C16 glycol ethers; alkanolamines; terpenes and mixtures thereof. Typically, the liquid composition herein may comprise up to 30%, preferably from 1% to 25%, more preferably from 1% to 20% and most preferably from 2%to 10% by weight of the total composition of said solvent or mixture thereof.

Because phase separation is a constant challenge for liquid detergent compositions, especially when the salt content in such compositions is high, the solvent system of the present invention is particularly designed to minimize

the risk of phase separation. Specifically, the solvent system of the present invention is composed mostly of diols, such as ethylene glycol, diethylene glycol, propylene glycol, dipropylene glycol, butylene glycol, pentanediols, and combinations thereof. The diols are present in the liquid detergent composition of the present invention in a total amount ranging from about 2 wt% to about 50 wt%. Preferably, the composition contains ethylene, diethylene glycol, and/or propylene glycol in a total amount ranging from about 5 wt% to about 40 wt%. More preferably, the composition contains propylene glycol in the amount ranging from about 15 wt% to about 35 wt%. Other organic solvents may also be present, which include, but are not limited to: methanol, ethanol, glycerin, sodium cumene sulfonate, potassium cumene sulfonate, ammonium cumene sulfonate, sodium toluene sulfonate, potassium toluene sulfonate, sodium xylene sulfonate, potassium xylene sulfonate, ammonium xylene sulfonate, or mixtures thereof. Other lower alcohols, such $C_1$-$C_4$ alkanolamines, e.g., monoethanolamine and/or triethanolamine, may also be used. In a particularly preferred embodiment of the present invention, the liquid detergent compositions of the present invention also contain from about 5 wt% to about 20 wt%, preferably from 6 wt% to 18 wt%, more preferably from 8 wt% to 16 wt% of glycerin in addition to the diol(s).

The liquid detergent composition of the present invention preferably contains water in combination with the above-mentioned organic solvent(s) as carrier(s). In some embodiments, water is present in the liquid detergent compositions of the present invention in the amount ranging from about 20 wt% to about 90 wt%, preferably from about 25 wt% to 80 wt%, and more preferably from about 30 wt% to about 70 wt%. In other embodiments, water is absent and the composition is anhydrous. Highly preferred compositions afforded by the present invention are clear, isotropic liquids. The liquid detergent composition as described herein above may also contain an external structurant, which may be present in an amount ranging from about 0.001% to about 1.0%, preferably from about 0.05% to about 0.5%, more preferably from about 0.1% to about 0.3% by total weight of the composition. Particularly preferred external structurants for the practice of the present invention are selected from hydrogenated castor oil, which is also referred to as trihydroxylstearin and is commercially available under the tradename Thixin®, and optionally modified natural fibres such as citrus fibres.

The balance of the cleaning composition of the present invention typically contains from about 5 wt% to about 70 wt%, or about 10 wt% to about 60 wt% adjunct ingredients.

Suitable adjunct ingredients for laundry detergent products include: builders, chelating agents, dye transfer inhibiting agents, dispersants, rheology modifiers, catalytic materials, bleach activators, hydrogen peroxide, sources of hydrogen peroxide, preformed peracids, polymeric dispersing agents, clay soil removal/anti-redeposition agents, brighteners, suds suppressors, dyes, photobleaches, structure elasticizing agents, fabric softeners, carriers, hydrotropes, processing aids, solvents, hueing agents, anti-microbial agents, free perfume oils, pungent agents, aversive agents, emetic agents, bittering agents and/or pigments. The precise nature of these adjunct ingredients and the levels thereof in the liquid laundry detergent composition will depend on factors like the specific type of the composition and the nature of the cleaning operation for which it is to be used.

Suitable adjunct ingredients for dish detergent products include: builders, chelants, conditioning polymers, cleaning polymers, surface modifying polymers, soil flocculating polymers, structurants, emollients, humectants, skin rejuvenating actives, carboxylic acids, scrubbing particles, bleach and bleach activators, perfumes, malodor control agents, pigments, dyes, opacifiers, beads, pearlescent particles, microcapsules, organic and inorganic cations such as alkaline earth metals such as Ca/Mg-ions and diamines, antibacterial agents, preservatives and pH adjusters and buffering means.

When the composition comprises a solid free-flowing particulate detergent composition preferably comprises a fully formulated laundry detergent composition, not a portion thereof such as a spray-dried, extruded or agglomerate particle that only forms part of the laundry detergent composition. Typically, the solid composition comprises a plurality of chemically different particles, such as spray-dried base detergent particles and/or agglomerated base detergent particles and/or extruded base detergent particles, in combination with one or more, typically two or more, or five or more, or even ten or more particles selected from: surfactant particles, including surfactant agglomerates, surfactant extrudates, surfactant needles, surfactant noodles, surfactant flakes; phosphate particles; zeolite particles; silicate salt particles, especially sodium silicate particles; carbonate salt particles, especially sodium carbonate particles; polymer particles such as carboxylate polymer particles, cellulosic polymer particles, starch particles, polyester particles, polyamine particles, terephthalate polymer particles, polyethylene glycol particles; aesthetic particles such as coloured noodles, needles, lamellae particles and ring particles; enzyme particles such as protease granulates, amylase granulates, lipase granulates, cellulase granulates, mannanase granulates, pectate lyase granulates, xyloglucanase granulates, bleaching enzyme granulates and co- granulates of any of these enzymes, preferably these enzyme granulates comprise sodium sulphate; bleach particles, such as percarbonate particles, especially coated percarbonate particles, such as percarbonate coated with carbonate salt, sulphate salt, silicate salt, borosilicate salt, or any combination thereof, perborate particles, bleach activator particles such as tetra acetyl ethylene diamine particles and/or alkyl oxybenzene sulphonate particles, bleach catalyst particles such as transition metal catalyst particles, and/or isoquinolinium bleach catalyst particles, pre-formed peracid particles, especially coated pre-formed peracid particles; filler particles such as sulphate salt particles and chloride particles; clay particles such as montmorillonite particles and particles of clay and

silicone; flocculant particles such as polyethylene oxide particles; wax particles such as wax agglomerates; silicone particles, brightener particles; dye transfer inhibition particles; dye fixative particles; perfume particles such as perfume microcapsules and starch encapsulated perfume accord particles, or pro-perfume particles such as Schiff base reaction product particles; hueing dye particles; chelant particles such as chelant agglomerates; and any combination thereof.

Polymers

Carboxylate polymer: The composition may comprise a carboxylate polymer, such as a maleate/acrylate random copolymer or polyacrylate homopolymer. Suitable carboxylate polymers include: polyacrylate homopolymers having a molecular weight of from 4,000 Da to 9,000 Da; maleate/acrylate random copolymers having a molecular weight of from 50,000 Da to 100,000 Da, or from 60,000 Da to 80,000 Da.

Another suitable carboxylate polymer is a co-polymer that comprises: (i) from 50 to less than 98 wt% structural units derived from one or more monomers comprising carboxyl groups; (ii) from 1 to less than 49 wt% structural units derived from one or more monomers comprising sulfonate moieties; and (iii) from 1 to 49 wt% structural units derived from one or more types of monomers selected from ether bond-containing monomers represented by formulas (I) and (II):

formula (I):

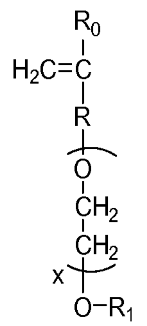

wherein in formula (I), $R_0$ represents a hydrogen atom or $CH_3$ group, R represents a $CH_2$ group, $CH_2CH_2$ group or single bond, X represents a number 0-5 provided X represents a number 1-5 when R is a single bond, and $R_1$ is a hydrogen atom or $C_1$ to $C_{20}$ organic group;

formula (II)

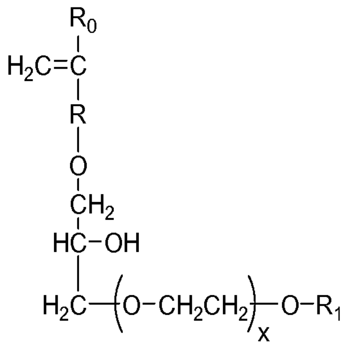

wherein in formula (II), $R_0$ represents a hydrogen atom or $CH_3$ group, R represents a $CH_2$ group, $CH_2CH_2$ group or single bond, X represents a number 0-5, and $R_1$ is a hydrogen atom or $C_1$ to $C_{20}$ organic group.

It may be preferred that the polymer has a weight average molecular weight of at least 50kDa, or even at least 70kDa.

Soil release polymer: The composition may comprise a soil release polymer. A suitable soil release polymer has a structure as defined by one of the following structures (I), (II) or (III):

(I) $-[(OCHR^1-CHR^2)_a-O-OC-Ar-CO-]_d$

(II) $-[(OCHR^3-CHR^4)_b-O-OC-sAr-CO-]_e$

(III) $-[(OCHR^5-CHR^6)_c-OR^7]_f$

wherein:

- a, b and c are from 1 to 200;
- d, e and f are from 1 to 50;
- Ar is a 1,4-substituted phenylene;
- sAr is 1,3-substituted phenylene substituted in position 5 with $SO_3Me$;
- Me is Li, K, Mg/2, Ca/2, Al/3, ammonium, mono-, di-, tri-, or tetraalkylammonium wherein the alkyl groups are $C_1$-$C_{18}$ alkyl or $C_2$-$C_{10}$ hydroxyalkyl, or mixtures thereof;
- $R^1$, $R^2$, $R^3$, $R^4$, $R^5$ and $R^6$ are independently selected from H or $C_1$-$C_{18}$ n- or iso-alkyl; and
- $R^7$ is a linear or branched $C_1$-$C_{18}$ alkyl, or a linear or branched $C_2$-$C_{30}$ alkenyl, or a cycloalkyl group with 5 to 9 carbon atoms, or a $C_8$-$C_{30}$ aryl group, or a $C_6$-$C_{30}$ arylalkyl group.

Suitable soil release polymers are sold by Clariant under the TexCare® series of polymers, e.g. TexCare® SRN240 and TexCare® SRA300. Other suitable soil release polymers are sold by Solvay under the Repel-o-Tex® series of polymers, e.g. Repel-o-Tex® SF2 and Repel-o-Tex® Crystal.

Anti-redeposition polymer: Suitable anti-redeposition polymers include polyethylene glycol polymers and/or polyethyleneimine polymers.

Suitable polyethylene glycol polymers include random graft co-polymers comprising: (i) hydrophilic backbone comprising polyethylene glycol; and (ii) hydrophobic side chain(s) selected from the group consisting of: $C_4$-$C_{25}$ alkyl group, polypropylene, polybutylene, vinyl ester of a saturated $C_1$-$C_6$ mono-carboxylic acid, $C_1$-$C_6$ alkyl ester of acrylic or methacrylic acid, and mixtures thereof. Suitable polyethylene glycol polymers have a polyethylene glycol backbone with random grafted polyvinyl acetate side chains. The average molecular weight of the polyethylene glycol backbone can be in the range of from 2,000 Da to 20,000 Da, or from 4,000 Da to 8,000 Da. The molecular weight ratio of the polyethylene glycol backbone to the polyvinyl acetate side chains can be in the range of from 1:1 to 1:5, or from 1:1.2 to 1:2. The average number of graft sites per ethylene oxide units can be less than 1, or less than 0.8, the average number of graft sites per ethylene oxide units can be in the range of from 0.5 to 0.9, or the average number of graft sites per ethylene oxide units can be in the range of from 0.1 to 0.5, or from 0.2 to 0.4. A suitable polyethylene glycol polymer is Sokalan HP22.

Cellulosic polymer: Suitable cellulosic polymers are selected from alkyl cellulose, alkyl alkoxyalkyl cellulose, carboxyalkyl cellulose, alkyl carboxyalkyl cellulose, sulphoalkyl cellulose, more preferably selected from carboxymethyl cellulose, methyl cellulose, methyl hydroxyethyl cellulose, methyl carboxymethyl cellulose, and mixures thereof.

Suitable carboxymethyl celluloses have a degree of carboxymethyl substitution from 0.5 to 0.9 and a molecular weight from 100,000 Da to 300,000 Da.

Suitable carboxymethyl celluloses have a degree of substitution greater than 0.65 and a degree of blockiness greater than 0.45, e.g. as described in WO09/154933.

Care polymers: Suitable care polymers include cellulosic polymers that are cationically modified or hydrophobically modified. Such modified cellulosic polymers can provide anti-abrasion benefits and dye lock benefits to fabric during the laundering cycle. Suitable cellulosic polymers include cationically modified hydroxyethyl cellulose.

Other suitable care polymers include dye lock polymers, for example the condensation oligomer produced by the condensation of imidazole and epichlorhydrin, preferably in ratio of 1:4:1. A suitable commercially available dye lock polymer is Polyquart® FDI (Cognis).

Other suitable care polymers include amino-silicone, which can provide fabric feel benefits and fabric shape retention benefits.

Bleach: Suitable bleach includes sources of hydrogen peroxide, bleach activators, bleach catalysts, pre-formed peracids and any combination thereof. A particularly suitable bleach includes a combination of a source of hydrogen peroxide with a bleach activator and/or a bleach catalyst.

Source of hydrogen peroxide: Suitable sources of hydrogen peroxide include sodium perborate and/or sodium percarbonate.

Bleach activator: Suitable bleach activators include tetra acetyl ethylene diamine and/or alkyl oxybenzene sulphonate.

Bleach catalyst: The composition may comprise a bleach catalyst. Suitable bleach catalysts include oxaziridinium bleach catalysts, transistion metal bleach catalysts, especially manganese and iron bleach catalysts. A suitable bleach catalyst has a structure corresponding to general formula below:

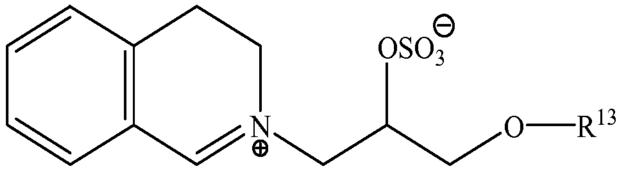

wherein $R^{13}$ is selected from the group consisting of 2-ethylhexyl, 2-propylheptyl, 2-butyloctyl, 2-pentylnonyl, 2-hexyldecyl, n-dodecyl, n-tetradecyl, n-hexadecyl, n-octadecyl, iso-nonyl, isodecyl, iso-tridecyl and iso-pentadecyl.

Pre-formed peracid: Suitable pre-form peracids include phthalimido-peroxycaproic acid.

Brightener: Suitable fluorescent brighteners include: di-styryl biphenyl compounds, e.g. Tinopal® CBS-X, diamino stilbene di-sulfonic acid compounds, e.g. Tinopal® DMS pure Xtra and Blankophor® HRH, and Pyrazoline compounds, e.g. Blankophor® SN, and coumarin compounds, e.g. Tinopal® SWN.

Preferred brighteners are: sodium 2 (4-styryl-3-sulfophenyl)-2H-napthol[1,2-d]triazole, disodium 4,4'-bis{[(4-anilino-6-(N methyl-N-2 hydroxyethyl)amino 1,3,5-triazin-2-yl)];amino}stilbene-2-2'disulfonate, disodium 4,4'-bis{[(4-anilino-6-morphofino-1,3,5-triazin-2-yl)]amino}stilbene-2-2'disulfonate, and disodium 4,4'- bis(2-sulfostyryl)biphenyl. A suitable fluorescent brightener is C.I. Fluorescent Brightener 260, which may be used in its beta or alpha crystalline forms, or a mixture of these forms.

Hueing agent: Suitable hueing agents include small molecule dyes, typically falling into the Colour Index (C.I.) classifications of Acid, Direct, Basic, Reactive (including hydrolysed forms thereof) or Solvent or Disperse dyes, for example classified as Blue, Violet, Red, Green or Black, and provide the desired shade either alone or in combination. Preferred such hueing agents include Acid Violet 50, Direct Violet 9, 66 and 99, Solvent Violet 13 and any combination thereof.

Suitable hueing agents may be alkoxylated. Such alkoxylated compounds may be produced by organic synthesis that may produce a mixture of molecules having different degrees of alkoxylation. Such mixtures may be used directly to provide the hueing agent, or may undergo a purification step to increase the proportion of the target molecule. Suitable hueing agents include alkoxylated bis-azo dyes.

The hueing agent may be incorporated into the detergent composition as part of a reaction mixture which is the result of the organic synthesis for a dye molecule, with optional purification step(s). Such reaction mixtures generally comprise the dye molecule itself and in addition may comprise un-reacted starting materials and/or by-products of the organic synthesis route.

Dye transfer inhibitors: Suitable dye transfer inhibitors include polyamine N-oxide polymers, copolymers of N-vinylpyrrolidone and N-vinylimidazole, polyvinylpyrrolidone, polyvinyloxazolidone, polyvinylimidazole and mixtures thereof. Preferred are poly(vinyl pyrrolidone), poly(vinylpyridine betaine), poly(vinylpyridine N-oxide), poly(vinyl pyrrolidone-vinyl imidazole) and mixtures thereof. Suitable commercially available dye transfer inhibitors include PVP-K15 and K30 (Ashland), Sokalan® HP165, HP50, HP53, HP59, HP56K, HP56, HP66 (BASF), Chromabond® S-400, S403E and S-100 (Ashland).

Perfume: Suitable perfumes comprise perfume materials selected from the group: (a) perfume materials having a ClogP of less than 3.0 and a boiling point of less than 250°C (quadrant 1 perfume materials); (b) perfume materials having a ClogP of less than 3.0 and a boiling point of 250°C or greater (quadrant 2 perfume materials); (c) perfume materials having a ClogP of 3.0 or greater and a boiling point of less than 250°C (quadrant 3 perfume materials); (d) perfume materials having a ClogP of 3.0 or greater and a boiling point of 250°C or greater (quadrant 4 perfume materials); and (e) mixtures thereof.

It may be preferred for the perfume to be in the form of a perfume delivery technology. Such delivery technologies further stabilize and enhance the deposition and release of perfume materials from the laundered fabric. Such perfume delivery technologies can also be used to further increase the longevity of perfume release from the laundered fabric. Suitable perfume delivery technologies include: perfume microcapsules, pro-perfumes, polymer assisted deliveries, molecule assisted deliveries, fiber assisted deliveries, amine assisted deliveries, cyclodextrin, starch encapsulated accord, zeolite and other inorganic carriers, and any mixture thereof.

A preferred detergent composition is preferably a manual dishwashing detergent, preferably in liquid form. It typically contains from 30% to 95%, preferably from 40% to 90%, more preferably from 50% to 85% by weight of a liquid carrier in which the other essential and optional components are dissolved, dispersed or suspended. One preferred component of the liquid carrier is water.

Preferably the pH of the detergent is adjusted to between 3 and 14, more preferably between 4 and 13, more preferably between 6 and 12 and most preferably between 8 and 10. The pH of the detergent can be adjusted using pH modifying ingredients known in the art.

Method of washing

Other aspects of the invention are directed to methods of washing ware or fabrics, especially dishware with the composition of the present invention. Said methods comprise the step of applying the composition, preferably in liquid form, onto the soiled surfaces especially dishware surface, either in diluted or neat form and rinsing or leaving the composition to dry on the surface without rinsing the surface.

By "in its neat form", it is meant herein that said composition is applied directly onto the surface to be treated and/or onto a cleaning device or implement such as a pre-treat device, dish cloth, a sponge or a dish brush without undergoing any dilution (immediately) prior to the application. The cleaning device or implement is preferably wet before or after the composition is delivered to it. By "diluted form", it is meant herein that said composition is diluted by the user with an appropriate solvent, typically water. By "rinsing", it is meant herein contacting the surface, such as the dishware cleaned using a process according to the present invention with substantial quantities of appropriate solvent, typically water, after the step of applying the liquid composition herein onto said dishware. By "substantial quantities", it is meant usually about 1 to about 10 liters.

The composition herein can be applied in its diluted form. Soiled surfaces e.g. dishes are contacted with an effective amount, typically from about 0.5 ml to about 20 ml (per about 25 dishes being treated), preferably from about 3ml to about 10 ml, of the detergent composition, preferably in liquid form, of the present invention diluted in water. The actual amount of detergent composition used will be based on the judgment of user, and will typically depend upon factors such as the particular product formulation of the composition, including the concentration of active ingredients in the composition, the number of soiled dishes to be cleaned, the degree of soiling on the dishes, and the like. Generally, from about 0.01 ml to about 150 ml, preferably from about 3ml to about 40ml of a liquid detergent composition of the invention is combined with from about 2000 ml to about 20000 ml, more typically from about 5000 ml to about 15000 ml of water in a sink having a volumetric capacity in the range of from about 1000 ml to about 20000 ml, more typically from about 5000 ml to about 15000 ml. The soiled dishes are immersed in the sink containing the diluted compositions then obtained, where contacting the soiled surface of the dish with a cloth, sponge, or similar article cleans them. The cloth, sponge, or similar article may be immersed in the detergent composition and water mixture prior to being contacted with the dish surface, and is typically contacted with the dish surface for a period of time ranged from about 1 to about 10 seconds, although the actual time will vary with each application and user. The contacting of cloth, sponge, or similar article to the surface is preferably accompanied by a concurrent scrubbing of the surface.

Another method of the present invention will comprise immersing the soiled surfaces such as dishes into a water bath or held under running water without any detergent composition. A device for absorbing detergent composition, such as a sponge or pre-treat device, is placed directly into a separate quantity of undiluted detergent composition, preferably in the form of a liquid for a period of time typically ranging from about 1 to about 5 seconds. The absorbing device, and consequently the undiluted detergent composition, is then contacted individually to the surface of each of the soiled dishes to remove said soiling. The absorbing device is typically contacted with each surface for a period of time range from about 1 to about 10 seconds, although the actual time of application will be dependent upon factors such as the degree of soiling of the surface. The contacting of the absorbing device to the soiled surface is preferably accompanied by concurrent scrubbing.

Alternatively, the device may be immersed in a mixture of the detergent composition and water prior to being contacted with the soiled surface, the concentrated solution is made by diluting the detergent composition with water in a small container that can accommodate the cleaning device at weight ratios ranging from about 95:5 to about 5:95, preferably about 80:20 to about 20:80 and more preferably about 70:30 to about 30:70, respectively, of detergent composition, preferably in liquid form the ratio of detergent composition:water respectively depending upon the user habits and the cleaning task. These methods are particularly applicable to soiled surfaces which are dishware. Preferably, the surfactant system concentration in the wash liquor is less than five, preferably less than ten times less than the critical micelle concentration of any of the surfactants of the surfactant system. More preferably, the concentration of the surfactant system in the wash liquor is less than the critical micelle concentration of any of the surfactants of the surfactant system. Critical micelle concentration (CMC) is defined as the concentration above which micelles form. At low surfactant concentration the surfactant molecules arrange on the surface. When more surfactant is added the surface tension of the solution starts to rapidly decrease since more and more surfactant molecules will be on the surface. When the surface becomes saturated, the addition of the surfactant molecules will lead to formation of micelles. This concentration point is called critical micelle concentration. The critical micelle concentration of the surfactant is determined by measuring surface tension of solutions using the Wilhelmy Plate method at room temperature (25°C) in distilled water, in accordance with ASTM D1331-14 method. Surface tension measurements are taken over a range of surfactant concentrations using a Kruss K100 Tensiometer, and the CMC determined from the point of inflection as illustrated in Figure 1.

The detergent composition according to the invention might also be used as a pretreating composition prior to the exposing the soiled items to an automatic washing machine. Following pretreatment, the soiled surface may be

washed in a washing machine or otherwise rinsed. In machine methods soiled surfaces may be treated with an aqueous wash liquor in which an effective amount of a cleaning composition of the invention is dissolved or dispensed into therein. An "effective amount" of the cleaning composition means from about 10g to about 300g of product dissolved or dispersed in a wash solution of volume from about 5L to about 65L. The water temperatures may range from about 5°C to about 100°C. The water to soiled material (e.g., fabric) ratio may be from about 1:1 to about 30:1. The compositions may be employed at concentrations of from about 500 ppm to about 15,000 ppm in solution. In the context of a fabric laundry composition, usage levels may also vary depending not only on the type and severity of the soils and stains, but also on the wash water temperature, the volume of wash water, and the type of washing machine (e.g., top-loading, front-loading, top-loading, vertical-axis Japanese-type automatic washing machine).

The present invention is directed to manual washing methods or hand washing/soak methods, and combined manualwashing with semi-automatic washing machines, are also included. Temperatures are typically lower, below 50, 45, 40, 35, 30, or 25°C.

EXAMPLES

Hereinafter, the present invention is described in more detail based on examples. All percentages are by weight unless otherwise specified.

Example 1. Exemplary Manual Dish-Washing Detergent Composition

| Level (as 100% active) | |
|---|---|
| Sodium alkyl ethoxy sulfate (C1213E00.6S) | 22.91% |
| n-C12-14 Di Methyl Amine Oxide | 7.64% |
| Lutensol XP80 (non-ionic surfactant supplied by BASF) | 0.45% |
| Sodium Chloride | 1.2% |
| Poly Propylene Glycol | 1% |
| Ethanol | 2% |
| Sodium Hydroxide | 0.24% |
| BslA protein | 0.05% |
| Minors (perfume, preservative, dye) + water | To 100 % |
| pH (@ 10% solution) | 9 |

Example 2

Exemplary Liquid Laundry Detergent Compositions

The following liquid laundry detergent compositions are prepared by traditional means known to those of ordinary skill in the art by mixing the following ingredients.

| Ingredients (wt%) | 2A | 2B | 2C |
|---|---|---|---|
| AES[1] | 17 | 2 | 11 |
| LAS[2] | 2.8 | 15 | 10 |
| AE[3] | 2.3 | 2.37 | 3.44 |
| Citric Acid | 5 | 1.98 | -- |
| Boric Acid | -- | 1 | 3 |
| Amine Oxide | 1.2 | -- | 0.5 |
| Trimethyl Lauryl Ammonium Chloride | -- | 1.5 | -- |
| PEI Polymer | 0.1~3.5 | 1 | 2 |
| Fatty Acids (substantially free of oleic acid) | 0.6 | 1.2 | 1.2 |

(continued)

| Ingredients (wt%) | 2A | 2B | 2C |
|---|---|---|---|
| Protease (54.5 mg/g)[4] | 7.62 | 7.98 | 2.08 |
| Amylase (29.26 mg/g)[5] | 2.54 | 2.67 | 0.69 |
| Xyloglucanase[6] | -- | -- | 0.15 |
| BslA protein | 0.1 | 0.1 | 0.05 |
| Borax | 4.72 | 4.94 | -- |
| Calcium Formate | 0.15 | 0.16 | 0.16 |
| Amphiphilic polymer[7] | -- | 1.5 | 4.36 |
| Hexamethylene diamine, ethoxylated, quaternized, sulfated[8] | -- | -- | 1.68 |
| DTPA[9] (50% active) | 0.28 | 0.3 | 0.64 |
| Tiron® | 0.84 | 0.89 | -- |
| Optical Brightener[10] | 0.34 | 0.37 | 0.36 |
| Ethanol | 0.97 | 4.1 | 2.99 |
| Propylene Glycol | 4.9 | 5.16 | 8.49 |
| Diethylene Glycol | -- | -- | 4.11 |
| Monoethanolamine (MEA) | 1.12 | 1.17 | 0.23 |
| Caustic Soda (NaOH) | 3.5 | 3.74 | 2.1 |
| Na Formate | 0.61 | 0.64 | 0.23 |
| Na Cumene Sulfonate | -- | -- | 1 |
| Suds Suppressor | -- | -- | 0.18 |
| Dye | 0.01 | -- | 0.02 |
| Perfume | 0.85 | -- | 1 |
| Preservative[11] | 0.05 | 0.5 | -- |
| Hydrogenated castor oil | -- | -- | 0.27 |
| Water | Q.S. | Q.S. | Q.S. |

Example 3

Exemplary Liquid Detergent Compositions for Use in Unit Dose (UP) Products

The following liquid detergent compositions are prepared and encapsulated in a multi-compartment pouch formed by a polyvinyl alcohol-film.

TABLE 6

| | A | B |
|---|---|---|
| Usage (g) | 25.36 | 24.34 |
| Usage (ml) | 23.7 | 22.43 |
| Wash Volume (L) | 64 | 64 |
| Anionic/Nonionic ratio | 1.73 | 9.9 |
| | | |

(continued)

| **Ingredients** (wt%) | | |
|---|---|---|
| Linear $C_9$-$C_{15}$ Alkylbenzene sulfonic acid | 18.25 | 22.46 |
| HC24/25 AE2/3S 90/10 blend | 8.73 | 15.29 |
| $C_{12-14}$ alkyl 9-ethoxylate | 15.56 | 3.82 |
| Citric Acid | 0.65 | 1.55 |
| Fatty acid (substantially free of unsaturated fatty acid) | 6.03 | 6.27 |
| Chelants | 1.16 | 0.62 |
| PEI Polymers | 1~6 | 3 |
| S Copolymers | 1~6 | 3 |
| Enzymes | 0.11 | 0.12 |
| BslA protein | 0.1 | 0.05 |
| Brightener 49 | 0.18 | 0.19 |
| Structurant | 0.1 | 0.1 |
| Solvent system* | 20.31 | 17.96 |
| Water | 10.31 | 11.66 |
| Perfume | 1.63 | 1.7 |
| Aesthetics | 1.48 | 1.13 |
| Mono-ethanolamine or NaOH (or mixture thereof) | 6.69 | 9.75 |
| Other laundry adjuncts / minors | Q.S. | Q.S. |
| *May include, but not limited to propanediol, glycerol, ethanol, dipropyleneglycol, polyetheyleneglycol, polypropyleneglycol. | | |

Example 4: Granular laundry detergent compositions for hand washing or washing machines, typically top-loading washing machines.

| Ingredient | 4A | 4B | 4C | 4D | 4E | 4F |
|---|---|---|---|---|---|---|
| | % weight | | | | | |
| LAS[2] | 11.33 | 10.81 | 7.04 | 4.20 | 3.92 | 2.29 |
| $C_{12-14}$Dimethylhydroxyethyl ammonium chloride | 0.70 | 0.20 | 1.00 | 0.60 | - | - |
| AES[1] | 0.51 | 0.49 | 0.32 | - | 0.08 | 0.10 |
| AE[3] | 8.36 | 11.50 | 12.54 | 11.20 | 16.00 | 21.51 |
| Sodium Tripolyphosphate | 5.0 | - | 4.0 | 9.0 | 2.0 | - |
| Zeolite A | - | 1.0 | - | 1.0 | 4.0 | 1.0 |
| Sodium silicate 1.6R | 7.0 | 5.0 | 2.0 | 3.0 | 3.0 | 5.0 |
| Sodium carbonate | 20.0 | 17.0 | 23.0 | 14.0 | 14.0 | 16.0 |
| Polyacrylate MW 4500 | 1.0 | 0.6 | 1.0 | 1.0 | 1.5 | 1.0 |
| Polymer grafted with vinyl acetate side chains[7] | 0.1 | 0.2 | - | - | 0.1 | - |
| Carboxymethyl cellulose | 1.0 | 0.3 | 1.0 | 1.0 | 1.0 | 1.0 |
| Acid Violet 50 | 0.05 | - | 0.02 | - | 0.04 | - |

(continued)

| Ingredient | 4A | 4B | 4C | 4D | 4E | 4F |
|---|---|---|---|---|---|---|
| | % weight | | | | | |
| Violet DD thiophene azo dye (Milliken) | - | 0.03 | - | 0.03 | - | 0.03 |
| Protease[4] | 0.10 | 0.10 | 0.10 | 0.10 | - | 0.10 |
| Amylase[5] | 0.03 | - | 0.03 | 0.03 | 0.03 | 0.03 |
| Lipase (Lipex from Novozymes) | 0.03 | 0.07 | 0.30 | 0.10 | 0.07 | 0.40 |
| Cellulase (Celluclean from Novozymes) | 0.002 | - | 0.05 | - | 0.02 | - |
| BslA protein | 0.1 | 0.1 | 0.05 | 0.08 | 0.2 | 0.02 |
| Optical Brightener[15] | 0.300 | 0.011 | 0.370 | 0.850 | 0.10 | 0.710 |
| Chelant[13] | 0.60 | 0.80 | 0.60 | 0.25 | 0.60 | 0.60 |
| DTI[12] | 0.62 | 0.35 | 0.15 | 0.30 | 0.20 | 0.40 |
| Sodium Percarbonate | - | 5.2 | 0.1 | - | - | - |
| Sodium Perborate | 4.4 | - | 3.85 | 2.09 | 0.78 | 3.63 |
| Nonanoyloxy benzensulphonate | 1.9 | 0.0 | 1.66 | 0.0 | 0.33 | 0.75 |
| Tetraacetylethylenediamine | 0.58 | 1.2 | 0.51 | 0.0 | 0.015 | 0.28 |
| Photobleach | 0.0030 | 0.0 | 0.0012 | 0.0030 | 0.0021 | - |
| S-ACMC[14] | 0.1 | 0.0 | 0.0 | 0.0 | 0.06 | 0.0 |
| Sulfate/Moisture | Balance | | | | | |

[1] AES can be $AE_{1.5}S$, $AE_2S$, and/or $AE_3S$, in the amount ranging from 0-20%.
[2] LAS can be provided in the amount ranging from 0-20%.
[3] AE is a C12-14 alcohol ethoxylate, with an average degree of ethoxylation of 7-9, supplied by Huntsman, Salt Lake City, Utah, USA. It can be provided in the amount ranging from 0-10%.
[4] Proteases may be supplied by Genencor International, Palo Alto, California, USA (e.g., Purafect Prime®, Excellase®) or by Novozymes, Bagsvaerd, Denmark (e.g. Liquanase®, Coronase®).
[5] Available from Novozymes, Bagsvaerd, Denmark (e.g., Natalase®, Mannaway®).
[6] Available from Novozymes (e.g., Whitezyme®).
[7] Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units, available from BASF as Sokalan PG101®.
[8] A compound having the following general structure: $bis((C_2H_5O)(C_2H_4O)_n)(CH_3)-N^+-C_xH_{2x}-N^+-(CH_3)-bis((C_2H_5O)(C_2H_4O)_n)$, wherein n = from 20 to 30, and x = from 3 to 8, or sulphated or sulphonated variants thereof, available from BASF as Lutenzit Z 96®
[9] DTPA is diethylenetriaminepentaacetic acid supplied by Dow Chemical, Midland, Michigan, USA.
[10] Suitable Fluorescent Whitening Agents are for example, Tinopal® AMS, Tinopal® CBS-X, Sulphonated zinc phthalocyanine Ciba Specialty Chemicals, Basel, Switzerland. It can be provided in the amount ranging from 0-5%.
[11] Suitable preservatives include methylisothiazolinone (MIT) or benzisothiazolinone (BIT), which can be provided in the amount ranging from 0-1%.
[12] DTI is poly(4-vinylpyridine-1-oxide) (such as Chromabond S-403E®) and/or poly(1-vinylpyrrolidone-co-1-vinylimidazole) (such as Sokalan HP56®).
[13] Chelant is diethylene triamine pentaacetic acid, 1-hydroxyethane 1,1-diphosphonic acid and/or sodium salt of ethylenediamine-N,N'-disuccinic acid, (S,S) isomer (EDDS)
[14] S-ACMC is Rective Blue 19 Azo-CM-Cellulose provided by Megazyme [15] Optical brightener is disodium 4,4'-bis{[4-anilino-6-morpholino-s-triazin-2-yl]-amino}-2,2'-stilbenedisulfonate, disodium 4,4'-bis-(2-sulfostyryl)biphenyl (sodium salt) and/or Optiblanc SPL10® from 3V Sigma

All percentages and ratios given for enzymes are based on active protein. All percentages and ratios herein

are calculated by weight unless otherwise indicated. All percentages and ratios are calculated based on the total composition unless otherwise indicated. It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

**Claims**

1. A detergent composition comprising a BslA protein and from 1 to 60% by weight of the composition of a surfactant system, wherein the surfactant system comprises an amphoteric and/or a zwitterionic surfactant in addition to an anionic surfactant and wherein the anionic surfactant and the amphoteric and/or the zwitterionic surfactant are in a weight ratio of less than 9:1, more preferably from 5:1 to 1:1, more preferably from 4:1 to 2:1, wherein the composition is a manual dishwashing composition.

2. A detergent composition according to claim 1 wherein the BslA protein has at least 80% identity to one of more of the following wild-type proteins: Bacillus subtilis BslA (SEQ ID NO:1), Bacillus licheniformis BslA (SEQ ID NO:2), Bacillus amyloliquefaciens BslA (SEQ ID NO:3), Bacillus pumilus BslA (SEQ ID NO:4), Bacillus subtilis Ywe A (SEQ ID NO:5).

3. A detergent composition according to any of claims 1 or 2 wherein the BslA protein has at least 90% identity to one of more of the following wild-type proteins: Bacillus subtilis BslA (SEQ ID NO:1), Bacillus licheniformis BslA (SEQ ID NO:2), Bacillus amyloliquefaciens BslA (SEQ ID NO:3), Bacillus pumilus BslA (SEQ ID NO:4), Bacillus subtilis Ywe A (SEQ ID NO:5).

4. A detergent composition according to any preceding claim wherein the BslA protein has at least 98% identity to one of more of the following wild-type proteins: Bacillus subtilis BslA (SEQ ID NO:1), Bacillus licheniformis BslA (SEQ ID NO:2), Bacillus amyloliquefaciens BslA (SEQ ID NO:3), Bacillus pumilus BslA (SEQ ID NO:4), Bacillus subtilis Ywe A (SEQ ID NO:5).

5. A detergent composition according to any preceding claim wherein the BslA protein is present in an amount from 0.0001 to 5% by weight of the composition, preferably from 0.001 to 1% by weight of the composition based on active protein, .

6. A composition according to any preceding claim wherein the surfactant system comprises a non-ionic surfactant.

7. A composition according to claim 1 wherein the amphoteric surfactant comprises an amine oxide surfactant and the zwitterionic surfactant comprises a betaine surfactant.

8. A composition according to any preceding claim additionally comprising an enzyme preferably selected from the group consisting of amylase, lipase, protease, cellulose and mixtures thereof.

9. A composition according to any preceding claim additionally comprising a chelant, preferably selected from amino carboxylate such as MGDA, GLDA and mixtures thereof or amino phosphonate chelant.

10. A method of manually washing soiled articles, preferably dishware, comprising the step of: delivering a composition according to any preceding claim to a volume of water to form a wash liquor and immersing the soiled articles, preferably dishware or fabric, in the liquor.

11. A method of manually washing soiled articles, preferably dishware, comprising the step of: delivering a composition according to any of claims 1 to 9 directly onto the soiled articles, preferably dishware, or onto a cleaning implement and using the cleaning implement to clean the articles, preferably dishware or fabric.

12. Use of a BslA protein to confer surface modification during cleaning.

**Patentansprüche**

1. Waschmittelzusammensetzung, umfassend ein BslA-Protein und von 1 bis 60 Gew.-% der Zusammensetzung ein Tensidsystem, wobei das Tensidsystem zusätzlich zu einem anionischen Tensid ein amphoteres und/oder ein zwitterionisches Tensid umfasst und wobei das anionische Tensid und das amphotere und/oder das zwitterionische Tensid in einem Gewichtsverhältnis von weniger als 9:1, mehr bevorzugt von 5:1 bis 1:1, mehr bevorzugt von 4:1 bis 2:1, vorliegen.

2. Waschmittelzusammensetzung nach Anspruch 1, wobei das BslA-Protein wenigstens 80 % Identität mit einem oder mehreren der folgenden Wildtyp-Proteine aufweist: Bacillus subtilis BslA (SEQ ID NO:1), Bacillus licheniformis BslA (SEQ ID NO:2), Bacillus amyloliquefaciens BslA (SEQ ID NO:3), Bacillus pumilus BslA (SEQ ID NO:4), Bacillus subtilis Ywe A (SEQ ID NO:5).

3. Waschmittelzusammensetzung nach einem der Ansprüche 1 oder 2, wobei das BslA-Protein wenigstens 90 % Identität mit einem oder mehreren der folgenden Wildtyp-Proteine aufweist: Bacillus subtilis BslA (SEQ ID NO:1), Bacillus licheniformis BslA (SEQ ID NO:2), Bacillus amyloliquefaciens BslA (SEQ ID NO:3), Bacillus pumilus BslA (SEQ ID NO:4), Bacillus subtilis Ywe A (SEQ ID NO:5).

4. Waschmittelzusammensetzung nach einem der vorstehenden Ansprüche, wobei das BslA-Protein wenigstens 98 % Identität mit einem oder mehreren der folgenden Wildtyp-Proteine aufweist: Bacillus subtilis BslA (SEQ ID NO:1), Bacillus licheniformis BslA (SEQ ID NO:2), Bacillus amyloliquefaciens BslA (SEQ ID NO:3), Bacillus pumilus BslA (SEQ ID NO:4), Bacillus subtilis Ywe A (SEQ ID NO:5).

5. Waschmittelzusammensetzung nach einem der vorstehenden Ansprüche, wobei das BslA-Protein in einer Menge von 0,0001 bis 5 Gew.-% der Zusammensetzung, vorzugsweise von 0,001 bis 1 Gew.-% der Zusammensetzung, basierend auf aktivem Protein, vorliegt.

6. Zusammensetzung nach einem der vorstehenden Ansprüche, wobei das Tensidsystem ein nichtionisches Tensid umfasst.

7. Zusammensetzung nach Anspruch 1, wobei das amphotere Tensid ein Aminoxidtensid umfasst und das zwitterionische Tensid ein Betaintensid umfasst.

8. Zusammensetzung nach einem der vorstehenden Ansprüche, zusätzlich umfassend ein Enzym, vorzugsweise ausgewählt aus der Gruppe bestehend aus Amylase, Lipase, Protease, Cellulose und Mischungen davon.

9. Zusammensetzung nach einem der vorstehenden Ansprüche, zusätzlich umfassend einen Chelatbildner, vorzugsweise ausgewählt aus Aminocarboxylat, wie beispielsweise MGDA, GLDA und Mischungen davon, oder Aminophosphonatchelat.

10. Verfahren zum manuellen Waschen verschmutzter Artikel, vorzugsweise Geschirr, umfassend den Schritt des: Abgebens einer Zusammensetzung nach einem der vorstehenden Ansprüche an ein Wasservolumen, um eine Waschflotte zu bilden, und des Eintauchens der verschmutzten Artikel, vorzugsweise des Geschirrs oder Stoffs, in die Waschflotte.

11. Verfahren zum manuellen Waschen verschmutzter Artikel, vorzugsweise Geschirr, umfassend den Schritt des: Abgebens einer Zusammensetzung nach einem der Ansprüche 1 bis 9 direkt auf die verschmutzten Artikel, vorzugsweise auf Geschirr, oder auf eine Reinigungsvorrichtung, und des Verwendens der Reinigungsvorrichtung zum Reinigen der Artikel, vorzugsweise des Geschirrs oder Stoffs.

12. Verwendung eines BslA-Proteins, um während der Reinigung eine Oberflächenmodifikation zu verleihen.

## Revendications

1. Composition détergente comprenant une protéine BslA et de 1 à 60 %, en poids de la composition, d'un système tensioactif, dans laquelle le système tensioactif comprend un agent tensioactif amphotère et/ou un agent tensioactif zwittérionique en plus d'un agent tensioactif anionique et dans laquelle l'agent tensioactif anionique et l'agent tensioactif amphotère et/ou l'agent tensioactif zwittérionique sont dans un rapport pondéral inférieur à 9:1, plus préférablement de 5:1 à 1:1, plus préférablement de 4:1 à 2:1, dans laquelle la composition est une composition de lavage manuel de la vaisselle.

2. Composition détergente selon la revendication 1 dans laquelle la protéine BslA présente au moins 80 % d'identité avec une ou plusieurs des protéines de type sauvage suivantes : Bacillus subtilis BslA (SEQ ID NO:1), Bacillus licheniformis BslA (SEQ ID NO:2), Bacillus amyloliquefaciens BslA (SEQ ID NO:3), Bacillus pumilus BslA (SEQ ID NO:4), Bacillus subtilis Ywe A (SEQ ID NO:5).

3. Composition détergente selon l'une quelconque des revendications 1 ou 2 dans laquelle la protéine BslA présente au moins 90 % d'identité avec une ou plusieurs des protéines de type sauvage suivantes : Bacillus subtilis BslA (SEQ ID NO:1), Bacillus licheniformis BslA (SEQ ID NO:2), Bacillus amyloliquefaciens BslA (SEQ ID NO:3), Bacillus pumilus BslA (SEQ ID NO:4), Bacillus subtilis Ywe A (SEQ ID NO:5).

4. Composition détergente selon une quelconque revendication précédente dans laquelle la protéine BslA présente au moins 98 % d'identité avec une ou plusieurs des protéines de type sauvage suivantes : Bacillus subtilis BslA (SEQ ID NO:1), Bacillus licheniformis BslA (SEQ ID NO:2), Bacillus amyloliquefaciens BslA (SEQ ID NO:3), Bacillus pumilus BslA (SEQ ID NO:4), Bacillus subtilis Ywe A (SEQ ID NO:5).

5. Composition détergente selon une quelconque revendication précédente dans laquelle la protéine BslA est présente en une quantité allant de 0,0001 à 5 %, en poids de la composition, de préférence de 0,001 à 1 %, en poids de la composition, sur une base de protéine active.

**6.** Composition selon l'une quelconque des revendications précédentes dans laquelle le système tensioactif comprend un agent tensioactif non ionique.

**7.** Composition selon la revendication 1 dans laquelle l'agent tensioactif amphotère comprend un agent tensioactif d'oxyde d'amine et l'agent tensioactif zwittérionique comprend un agent tensioactif de bétaïne.

**8.** Composition selon l'une quelconque des revendications précédentes comprenant en outre une enzyme choisie de préférence dans le groupe constitué d'amylase, lipase, protéase, cellulose et des mélanges de celles-ci.

**9.** Composition selon l'une quelconque des revendications précédentes comprenant en outre un agent chélatant, choisi de préférence parmi un aminocarboxylate tel que MGDA, GLDA et des mélanges de ceux-ci ou un agent chélatant aminophosphonate.

**10.** Procédé de lavage manuel d'articles souillés, de préférence de la vaisselle, comprenant l'étape consistant à : distribuer une composition selon l'une quelconque des revendications précédentes à un volume d'eau pour former une liqueur de lavage et immerger les articles souillés, de préférence de la vaisselle ou un tissu, dans la liqueur.

**11.** Procédé de lavage manuel d'articles souillés, de préférence de la vaisselle, comprenant l'étape consistant à : distribuer une composition selon l'une quelconque des revendications 1 à 9 directement sur les articles souillés, de préférence de la vaisselle, ou sur un ustensile de nettoyage et utiliser l'ustensile de nettoyage pour nettoyer les articles, de préférence de la vaisselle ou un tissu.

**12.** Utilisation d'une protéine BslA pour conférer une modification de surface pendant le nettoyage.

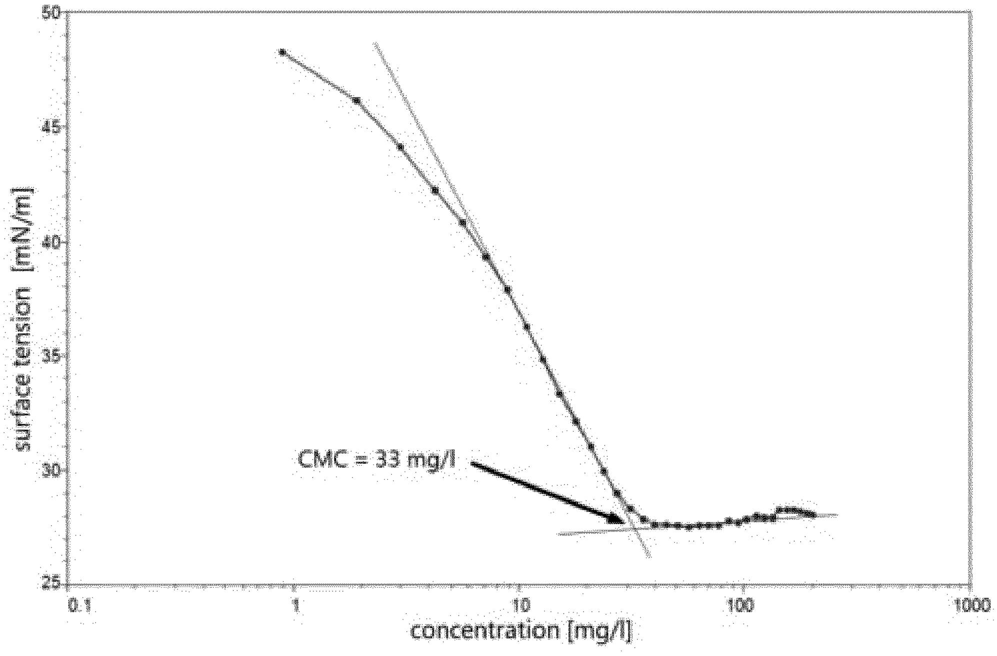
50
45
40
35
30
25
surface tension [mN/m]
CMC = 33 mg/l
0.1
1
10
100
1000
concentration [mg/l]

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9905243 A
- WO 9905242 A
- WO 9905244 A
- WO 9905082 A
- WO 9905084 A
- WO 9905241 A
- WO 9907656 A
- WO 0023549 A
- WO 0023548 A
- WO 06090335 A
- WO 13116261 A
- WO 2013171241 A
- WO 2011084412 A
- WO 2013033318 A
- US 5352604 A
- WO 11140316 A
- WO 11072117 A
- WO 06002643 A
- WO 201219844 A
- WO 201219849 A
- WO 201219848 A
- WO 09154933 A


### Non-patent literature cited in the description


- **NEEDLEMAN ; WUNSCH.** *J. Mol. Biol.,* 1970, vol. 48, 443-453
- **HENIKOFF S. ; HENIKOFF J.G.** *P.N.A.S. USA,* 1992, vol. 89, 10915-10919